(19) Europäisches Patentamt
European Patent Office
Office européen des brevets

(11) Publication number: 0 384 231 A1

# (12) EUROPEAN PATENT APPLICATION

(21) Application number: 90102526.2

(51) Int. Cl.5: C07D 231/46, A01N 43/56

(22) Date of filing: 09.02.90

(30) Priority: 21.02.89 DE 3905312

(43) Date of publication of application:
29.08.90 Bulletin 90/35

(84) Designated Contracting States:
BE CH DE FR GB IT LI NL

(71) Applicant: BAYER AG
D-5090 Leverkusen 1 Bayerwerk(DE)

(72) Inventor: Lunkenheimer, Winfried, Dr.
Bismarckstrasse 29
D-5600 Wuppertal 1(DE)
Inventor: Hänssler, Gerd, Dr.
Am Arenzberg 58a
D-5090 Leverkusen 3(DE)

(54) **Substituted 3-amino-2-pyrazolin-5-one derivatives, processes for their preparation, and their use for combatting parasites.**

(57) Substituierte 3-Amino-2-pyrazolin-5-on-Derivate der Formel (I)

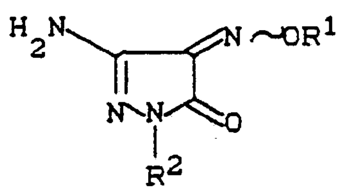

(I)

in welcher
$R^1$ und $R^2$ die in der Beschreibung gegebenen Bedeutungen haben, ihre Verwendung zur Bekämpfung von Schädlingen.

Die Verbindungen sind neu und allgemein durch die Formel (I) definiert. Sie können nach Analogieverfahren hergestellt werden, z.B. aus geeigneten 2-Cyan-2-oximinoessigsäure-Derivaten mit geeigneten Hydrazinen oder aus geeigneten 4-Oximino-2-pyrazolin-5-on-Derivaten mit geeigneten Alkylierungs- oder Acylierungsmitteln.

EP 0 384 231 A1

## Substituierte 3-Amino-2-pyrazolin-5-on-Derivate, Verfahren zu ihrer Herstellung und ihre Verwendung zur Bekämpfung von Schädlingen

Die vorliegende Erfindung betrifft neue substituierte 3-Amino-2-pyrazolin-5-on-Derivate, mehrere Verfahren zu ihrer Herstellung und ihre Verwendung in Schädlingsbekämpfungsmitteln, vor allem als Fungizide.

Es ist bereits bekannt, daß bestimmte substituierte 2-Pyrazolin-5-on-Derivate, wie beispielsweise 1,3-Dimethyl-4-(2-chlormethyl-benzyloximino)-2-pyrazolin-5-on und 1,3-Dimethyl-4-(4-phenyl-benzyloximino)-2-pyrazolin-5-on gute fungizide Wirkung besitzen (vgl. EP-OS 0 166 171). Die Wirkung dieser Verbindungen ist jedoch, insbesondere bei niedrigen Aufwandmengen und -konzentrationen, nicht immer voll befriedigend.

Es wurden neue substituierte 3-Amino-2-pyrazolin-5-on-Derivate der allgemeinen Formel (I)

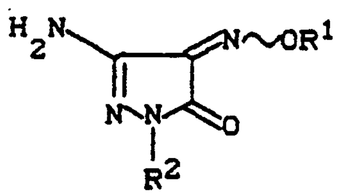

(I)

in welcher

$R^1$ für unsubstituiertes oder substituiertes Alkyl, unsubstituiertes oder substituiertes Alkenyl oder Alkinyl, unsubstituiertes oder substituiertes Cycloalkyl oder Cycloalkenyl steht,

$R^2$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl oder für einen der Reste

$-CX-YR^3$, $-CXNR^4R^5$, $-\underset{\underset{O}{\|}}{C}-R^6$,

$-SO_2NR^4R^5$ oder $-SO_2R^7$

steht,

$R^3$ für unsubstituiertes oder substituiertes Alkyl oder unsubstituiertes oder substituiertes Alkenyl oder Alkinyl steht,

$R^4$ und $R^5$ gleich oder verschieden sind und für Wasserstoff, unsubstituiertes oder substituiertes Alkyl, unsubstituiertes oder substituiertes Alkenyl oder Alkinyl, unsubstituiertes oder substituiertes Aryl oder Heterocyclyl oder gemeinsam mit dem Stickstoffatom, an welches sie gebunden sind, für einen unsubstituierten oder substituierten heterocyclischen Ring stehen, der weitere Heteroatome enthalten kann,

$R^6$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl, Alkoxycarbonyl oder Phenylcarbonyl steht,

$R^7$ für unsubstituiertes oder substituiertes Alkyl oder unsubstituiertes oder substituiertes Aryl steht und

X und Y gleich oder verschieden sind und für Sauerstoff oder Schwefel stehen,

gefunden.

Die Verbindungen der Formel (I) können als geometrische Isomere oder Isomerengemische unterschiedlicher Zusammensetzung vorliegen.

Sowohl die reinen Isomeren als auch die Isomerengemische werden erfindungsgemäß beansprucht.

Weiterhin wurde gefunden, daß man die neuen substituierten 3-Amino-2-pyrazolin-5-on-Derivate der allgemeinen Formel (I)

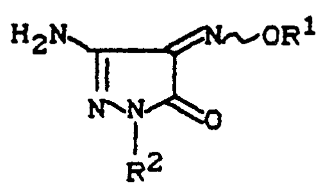

(I)

in welcher

$R^1$ für unsubstituiertes oder substituiertes Alkyl, unsubstituiertes oder substituiertes Alkenyl oder Alkinyl,

unsubstituiertes oder substituiertes Cycloalkyl oder Cycloalkenyl steht,

$R^2$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl oder für einen der Reste

$-CX-YR^3$, $-CXNR^4R^5$, $-\underset{\underset{O}{\Vert}}{C}-R^6$,

$-SO_2NR^4R^5$ oder $-SO_2R^7$

steht,

$R^3$ für unsubstituiertes oder substituiertes Alkyl oder unsubstituiertes oder substituiertes Alkenyl oder Alkinyl steht,

$R^4$ und $R^5$ gleich oder verschieden sind und für Wasserstoff, unsubstituiertes oder substituiertes Alkyl, unsubstituiertes oder substituiertes Alkenyl oder Alkinyl, unsubstituiertes oder substituiertes aryl oder Heterocyclyl oder gemeinsam mit dem Stickstoffatom, an welches sie gebunden sind, für einen unsubstituierten oder substituierten heterocyclischen Ring stehen, der weitere Heteroatome enthalten kann,

$R^6$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl, Alkoxycarbonyl oder Phenylcarbonyl steht,

$R^7$ für unsubstituiertes oder substituiertes Alkyl oder unsubstituiertes oder substituiertes Aryl steht und

X und Y gleich oder verschieden sind und für Sauerstoff oder Schwefel stehen,

erhält, wenn man

(a) 2-Cyan-2-oximino-essigsäure-Derivate der Formel (II)

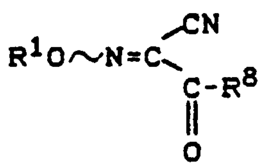

(II)

in welcher

$R^1$ die oben angegebene Bedeutung hat und

$R^8$ für niederes Alkoxy, vorzugsweise Methoxy oder Ethoxy oder Halogen, vorzugsweise Chlor steht,

mit Hydrazinen der Formel (III)

$H_2N-NH-R^2$ (III)

in welcher

$R^2$ die oben angegebene Bedeutung hat,

gegebenenfalls in Gegenwart eines Verdünnungsmittels, gegebenenfalls in Gegenwart einer Base und gegebenenfalls in Gegenwart eines Katalysators umsetzt oder wenn man

(b) die nach Verfahren (a) erhältlichen 4-Oximino-2-pyrazolin-5-on-Derivate der allgemeinen Formel (Ia)

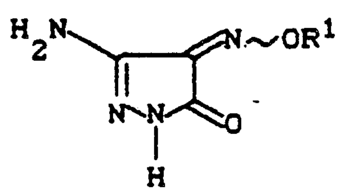

(Ia)

in welcher

$R^1$ die oben angegebene Bedeutung hat,

(b1) mit Mitteln der Formel (IV)

$R^{2-1}-A$ (IV)

in welcher

$R^{2-1}$ für unsubstituiertes oder substituiertes Alkyl, unsubstituiertes oder substituiertes Alkenyl oder Alkinyl, unsubstituiertes oder substituiertes Cycloalkyl, unsubstituiertes oder substituiertes Cycloalkenyl oder substituiertes oder unsubstituiertes Heterocyclyl steht und

A für eine elektronenanziehende Abgangsgruppe steht

oder

(b2) mit Acylierungsmitteln der Formel (Va)

$R^{2-2}$-Z (Va)

in welcher

$R^{2-2}$ für einen der Reste

$-CX-YR^3$, $-CXNR^4R^5$, $-\underset{\underset{O}{\|}}{C}-R^6$,

$-SO_2NR^4R^5$ oder

$-SO_2R^7$ steht,

Z für eine übliche Abgangsgruppe steht, wie Halogen, $-O-CO-R^{2-2}$, $-O-CO-OR^{2-1}$, $-OR^{2-1}$, $-SR^{2-1}$, Carboxymethoxy oder Carboxymethylthio und

$R^3$, $R^4$, $R^5$, $R^6$, $R^7$, X und Y die oben angegebene Bedeutung haben

oder

(b3) mit Acylierungsmitteln der Formel (Vb)

$R^{2-3}-N=C=X$ (Vb)

in welcher

$R^{2-3}$ für unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl oder substituiertes oder unsubstituiertes Heterocyclyl steht und

X für Sauerstoff oder Schwefel steht,

gegebenenfalls in Gegenwart eines Verdünnungsmittels, gegebenenfalls in Gegenwart einer Base und gegebenenfalls in Gegenwart eines Katalysators umsetzt;

oder wenn man

(c) 4-Oximino-2-pyrazolin-5-on-Derivate der Formel (VI)

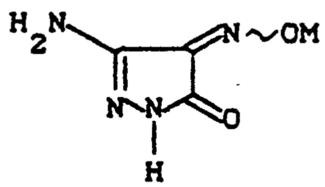

(VI)

in welcher

M für Wasserstoff oder ein Alkalimetallkation steht,

mit Alkylierungsmitteln der Formel (VII)

$R^1$-A (VII)

in welcher

$R^1$ die oben angegebene Bedeutung hat und

A für eine elektronenanziehende Abgangsgruppe steht,

gegebenenfalls in Gegenwart eines Verdünnungsmittels und gegebenenfalls in Gegenwart eines Katalysators umsetzt.

Schließlich wurde gefunden, daß die neuen substituierten 3-Amino-2-pyrazolin-5-on-Derivate der Formel (I) eine starke biologische Wirkung aufweisen. Dabei zeigen überraschenderweise die erfindungsgemäßen Verbindungen der Formel (I) z.B. eine bessere fungizide Wirkung als die aus dem Stand der Technik bekannten substituierten 2-Pyrazolin-5-on-Derivate, welche konstitutionell bzw. wirkungsmäßig naheliegende Verbindungen sind. Die erfindungsgemäßen Stoffe stellen somit eine Bereicherung der Technik dar.

Unsubstituiertes oder substituiertes Alkyl in der Definition von $R^1$ und $R^2$ und Alkyl in der Definition von $R^3$, $R^4$, $R^5$, $R^6$ und $R^7$ in den allgemeinen Formeln steht für geradkettiges oder verzweigtes Alkyl mit vorzugsweise 1 bis 10, besonders bevorzugt 1 bis 8 und ganz besonders bevorzugt 1 bis 6 Kohlenstoffatomen. Beispielhaft seien unsubstituiertes oder substituiertes Methyl, Ethyl, n-Propyl, i-Propyl, n-Butyl, sec-Butyl, i-Butyl, tert-Butyl, n-Pentyl, i-Pentyl und t-Pentyl genannt.

Unsubstituiertes oder substituiertes Alkenyl in den Definitionen von $R^1$ und $R^2$ und Alkenyl in der Definition von $R^3$, $R^4$, $R^5$ und $R^6$ in den allgemeinen Formeln steht für geradkettiges oder verzweigtes Alkenyl mit vorzugsweise 2 bis 8, besonders bevorzugt 2 bis 6 und insbesondere 2 bis 4, ganz besonders bevorzugt 3 Kohlenstoffatomen. Beispielhaft seien unsubstituiertes oder substituiertes Vinyl, Allyl, 2-Butenyl, 3-Butenyl und 1-Methallyl genannt.

Unter dem Begriff unsubstituiertes oder substituiertes Alkinyl in den Definitionen $R^1$ und $R^2$ und Alkinyl in der Definition von $R^3$, $R^4$, $R^5$ und $R^6$ in den allgemeinen Formeln ist geradkettiges oder verzweigtes

Alkinyl mit vorzugsweise 2 bis 6, insbesondere 2 bis 4, besonders bevorzugt 3 Kohlenstoffatomen zu verstehen. Beispielhaft seien unsubstituiertes oder substituiertes Ethinyl, 2-Propinyl, 2-Butinyl, 3-Butinyl und 1-Methyl-2-propinyl genannt.

Als unsubstituiertes oder substituiertes Cycloalkyl in den Definitionen $R^1$ und $R^2$ und Cycloalkyl in der Definition von $R^6$ steht Cycloalkyl mit vorzugsweise 3 bis 8, insbesondere 3, 5 oder 6 Kohlenstoffatomen.

Beispielhaft seien unsubstituiertes oder substituiertes Cyclopropyl, Cyclobutyl, Cyclopentyl, Cyclohexyl und Cycloheptyl genannt.

Als unsubstituiertes oder substituiertes Cycloalkenyl in den Definitionen von $R^1$ und $R^2$ und Cycloalkenyl in den Definitionen von $R^4$, $R^5$ und $R^6$ steht Cycloalkenyl mit vorzugsweise 5 oder 6, insbesondere 6 Kohlenstoffatomen.

Beispielhaft seien unsubstituiertes oder substituiertes Cyclopentenyl und Cyclohexenyl genannt.

Unter dem Begriff unsubstituiertes oder substituiertes Aryl in der Definition von $R^2$ in den allgemeinen Formeln und Aryl in der Definition von $R^4$, $R^5$, $R^6$ und $R^7$ ist Aryl mit vorzugsweise 6 bis 10 Kohlenstoffatomen im Arylteil zu verstehen. Beispielhaft seien gegebenenfalls substituiertes Phenyl oder Naphthyl, insbesondere Phenyl genannt.

Als unsubstituiertes oder substituiertes Heterocyclyl in der Definition von $R^2$ und Heterocyclyl oder heterocyclischer Ring in den Definitionen von $R^4$, $R^5$ und $R^6$ steht ein 5- oder 6-gliedriger Ring, der ein oder mehrere Heteroatome, bevorzugt 1 bis 3 gleiche oder verschiedene Heteroatome, enthält und die entsprechenden benzoanellierten Ringe. Als Heteroatome seien vorzugsweise Sauerstoff, Schwefel und Stickstoff genannt; beispielhaft seien genannt: Furanyl, Thienyl, Tetrahydrofuranyl, Thiolanyl, Pyridyl, Indolyl, N-Methylpyrrolyl, Pyrimidyl, Sulfolanyl, Morpholinyl, Thiazolyl, Benzothiazolyl, Thidiazolyl, Isoxazolyl, Pyrazinyl, Oxazolinyl, Pyrrolidinyl, Imidazolinyl, Piperidinyl, Thiomorpholinyl, 1,3-Oxazanyl, 1,3-Diazanyl.

Als unsubstituiertes oder substituiertes Alkoxycarbonyl in der Definition von $R^6$ steht geradkettiges oder verzweigtes Alkoxycarbonyl mit 1 bis 4, vorzugsweise 1 oder 2 Kohlenstoffatomen im Alkoxyrest; beispielhaft seien genannt: Methoxycarbonyl, Ethoxycarbonyl, n-Propoxycarbonyl, i-Propoxycarbonyl, n-, i-, sec- und t-Butoxycarbonyl.

Die substituierten Reste der allgemeinen Formeln können einen bis mehrere, vorzugsweise 1 bis 3, insbesondere 1 oder 2 gleiche oder verschiedene Substituenten tragen. Als Substituenten seien beispielhaft und vorzugsweise aufgeführt: Alkyl selbst oder als Bestandteil von Alkylcarbonyl, mit vorzugsweise 1 bis 4, insbesondere 1 oder 2 Kohlenstoffatomen, wie Methyl, Ethyl, n- oder i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i-, sec-und t-Butyloxy;

Halogen bedeutet im allgemeinen als Substituent vorzugsweise Fluor, Chlor, Brom und lod, insbesondere Fluor, Chlor und Brom und besonders bevorzugt Fluor und Chlor.

Unsubstituiertes oder substituiertes Cycloalkyl als Substituent steht für Cycloalkyl mit vorzugsweise 3 bis 8, insbesondere 3, 5 oder 6 Kohlenstoffatomen.

Beispielhaft seien gegebenenfalls substituiertes Cyclopropyl, Cyclobutyl, Cyclopentyl, Cyclohexyl und Cycloheptyl genannt.

Unsubstituiertes oder substituiertes Cycloalkenyl als Substituent steht für Cycloalkenyl mit vorzugsweise 5 oder 6, insbesondere 6 Kohlenstoffatomen.

Beispielhaft seien unsubstituiertes oder substituiertes Cyclopentenyl und Cyclohexenyl genannt.

Unsubstituiertes oder substituiertes Heterocyclyl als Substituent steht im allgemeinen für einen 5- oder 6-gliedrigen Ring, der ein oder mehrere Heteroatome, bevorzugt 1 bis 3 gleiche oder verschiedene Heteroatome, enthält. Als Heteroatome seien vorzugsweise Sauerstoff, Schwefel und Stickstoff genannt; beispielhaft seien genannt: Furanyl, Thienyl, Tetrahydrofuranyl, Thiolanyl, Pyrazolyl, 1,2,4-Triazolyl, Oxiranyl, 1,3-Dioxolanyl, Phthalimidyl, 2-Pyrrolidonyl, 3-Methyl-2-oxazolidinonyl, N-Methyl-pideridinyl, Tetrahydropyrrolyl, Morpholinyl, 1,3-Dioxanyl, 4-Methyl-thiazolyl, 4-Methyl-oxazolyl, 4,5-Dihydrooxazolyl, 4,5-Dihydro-thiazolyl, Pyrimidinyl, 1,2,4-Thiadiazolyl, 1,3,4-Thiadiazolyl und 1,3,4-Oxadiazolyl.

Speziell dieSubstituenten für Arylreste als solche oder in Zusammensetzungen wie Aryloxy, Aralkoxy und für die heterocyclischen Ringe haben die im folgenden angegebenen Bedeutungen.

Halogen steht im allgemeinen als Substituent der Reste für Fluor, Chlor, Brom und Jod, bevorzugt für Fluor, Chlor und Brom, besonders bevorzugt für Fluor und Chlor.

Alkyl steht im allgemeinen als Substituent der Reste für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkoxy steht im allgemeinen als Substituent der Reste für geradkettiges oder verzweigtes Alkoxy mit 1 bis 6, besonders bevorzugt 1 bis 3 Kohlenstoffatomen; beispielhaft seien genannt: Methoxy, Ethoxy, n- und

i-Propoxy, n-, i-, sec.- und t-Butoxy, n-Hexoxy und i-Hexoxy.

Halogenalkyl Halogenalkoxy stehen im allgemeinen als Substituenten in den Resten für geradkettige oder verzweigte Reste mit je 1 bis 4 Kohlenstoffatomen, besonders bevorzugt mit 1 oder 2 Kohlenstoffatomen und jeweils 1 bis 5 gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n- propyl, Difluormethyl, Trifluormethyl, Dichlormethyl, Trichlormethyl, Difluorethyl, Trifluorethyl, Trichlorethyl, Chlor-difluor-methyl, Trifluor-chlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Mono- oder Di-alkylamino steht für eine Aminogruppe mit 1 oder 2 Alkylgruppen, vorzugsweise 2 Alkylgruppen, welche jeweils geradkettig oder verzweigt sein können und vorzugsweise 1 bis 5, insbesondere 1 bis 3 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Die erfindungsgemäßen 3-Amino-2-pyrazolin-5-on-Derivate sind durch die Formel (I) allgemein definiert. In dieser Formel (I) stehen vorzugsweise

$R^1$ für unsubstituiertes oder ein- bis fünffach, gleich oder verschieden substituiertes, geradkettiges oder verzweigtes Alkyl mit 1 bis 10 Kohlenstoffatomen, wobei folgende Substituenten infrage kommen: Fluor, Chlor, Brom, Iod, Cyano, -COOH, -COOR$^3$, -CONR$^4$R$^5$, -OR$^6$, -SR$^6$, Alkylcarbonyl mit 1 bis 9 Kohlenstoffatomen, unsubstituiertes oder ein- bis dreifach, gleich oder verschieden durch Halogen substituiertes Phenylcarbonyl, unsubstituiertes oder ein- bis fünffach, gleich oder verschieden substituiertes Phenyl, wobei als Phenylsubstituenten genannt seien: Halogen, Nitro, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen; Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil; weitere Alkylsubstituenten sind unsubstituiertes oder ein- bis fünffach, gleich oder verschieden durch Chlor oder Alkyl mit 1 bis 4 Kohlenstoffatomen substituiertes Cycloalkyl oder Cycloalkenyl mit 3 bis 6 Kohlenstoffatomen, unsubstituiertes oder ein- bis fünffach, gleich oder verschieden durch Halogen, Hydroxy, Oxo, Alkyl und Alkoxy mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff-und Schwefelatomen; $R^1$ steht ferner vorzugsweise für jeweils unsubstituiertes oder ein-bis dreifach, gleich oder verschieden substituiertes, geradkettiges oder verzweigtes Alkenyl oder Alkinyl mit 2 bis 6 Kohlenstoffatomen, wobei als Substituenten genannt seien: unsubstituiertes oder ein- bis fünffach, gleich oder verschieden durch Halogen, Alkyl und Alkoxy mit 1 bis 4 Kohlenstoffatomen substituiertes Phenyl; $R^1$ steht außerdem vorzugsweise für jeweils unsubstituiertes oder ein- bis fünffach, gleich oder verschieden durch Chlor, Alkyl oder Alkoxy mit 1 bis 4 Kohlenstoffatomen substituiertes Cycloalkyl mit 3 bis 6 Kohlenstoffatomen oder Cycloalkenyl mit 5 bis 7 Kohlenstoffatomen;

$R^2$ für Wasserstoff, für unsubstituiertes oder ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, -COOH, -COOR$^3$, -CONR$^4$R$^5$, -OR$^6$, -SR$^6$, Alkylcarbonyl mit 1 bis 9 Kohlenstoffatomen, unsubstituiertes 1 oder ein- bis dreifach, gleich oder verschieden durch Halogen substituiertes Phenylcarbonyl, unsubstituiertes oder ein- bis fünffach, gleich oder verschieden substituiertes Phenyl, wobei als Phenylsubstituenten genannt seien: Halogen, Nitro, Alkyl und Alkoxy mit jeweils 1 bis 4 Kohlenstoffatomen, Halogenalkyl und Halogenalkoxy mit jeweils oder 2 Kohlenstoffatomen und 1 bis 5 gleichen oder verschiedenen Halogenatomen, Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil; weitere Alkylsubstituenten sind unsubstitu iertes oder ein- bis fünffach, gleich oder verschieden durch Chlor oder Alkyl mit 1 bis 4 Kohlenstoffatomen substituiertes Cycloalkyl mit 3 bis 6 Kohlenstoffatomen, unsubstituiertes oder ein- bis fünffach, gleich oder verschieden durch Halogen, Hydroxy, Oxo, Alkyl und Alkoxy mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff-und Schwefelatomen; $R^2$ steht ferner vorzugsweise für jeweils unsubstituiertes oder ein- bis dreifach, gleich oder verschieden substituiertes, geradkettiges oder verzweigtes Alkenyl oder Alkinyl mit 2 bis 6 Kohlenstoffatomen, wobei als Substituenten genannt seien: unsubstituiertes oder ein- bis fünffach, gleich oder verschieden durch Halogen und Alkyl mit 1 bis 4 Kohlenstoffatomen substituiertes Phenyl; $R^2$ steht ferner vorzugsweise für jeweils unsubstituiertes oder ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen: $R^2$ steht ferner für unsubstituiertes oder ein- bis fünffach, gleich oder verschieden substituiertes Aryl mit 6 bis 12 Kohlenstoffatomen, wobei als Substituenten genannt seien: Halogen, Nitro, Alkyl oder Alkoxy mit jeweils 1 bis 4 Kohlenstoffatomen, Halogenalkyl oder Halogenalkoxy mit jeweils 1 oder 2 Kohlenstofftomen im Alkylteil und 1 bis 5 gleichen oder verschiedenen Halogenatomen; $R^2$ steht ferner vorzugsweise für unsubstituiertes oder ein- bis fünffach, gleich oder verschieden durch Halogen, Hydroxy, Oxo, Alkyl und Alkoxy mit jeweils 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen oder für einen der Reste $-CX-YR^3$, $-CXNR^4R^5$, $-CO-R^6$, $-SO_2-NR^4R^5$ oder $-SO_2R^7$, worin X und Y gleich oder verschieden sind und für Sauerstoff oder Schwefel stehen,

$R^3$ für unsubstituiertes oder ein- bis fünffach, gleich oder verschieden substituiertes, geradkettiges oder verzweigtes Alkyl mit 1 bis 6 Kohlenstoffatomen, wobei als Substituenten genannt seien: Halogen, unsubstituiertes oder ein- bis fünffach, gleich oder verschieden substituiertes Phenyl, wobei als Phenylsubstituenten Halogen, Nitro, Alkyl und Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen, Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil infrage kommen und Cycloalkyl oder Cycloalkenyl mit 3 bis 6 Kohlenstoffatomen; $R^3$ ferner für geradkettiges oder verzweigtes Alkenyl oder Alkinyl mit 3 bis 6 Kohlenstoffatomen,

$R^4$ und $R^5$ jeweils gleich oder verschieden sind und für Wasserstoff oder für unsubstituiertes oder gleich oder verschieden, ein- oder zweifach substituiertes Alkyl mit 1 bis 6 Kohlenstoffatomen, wobei folgende Substituenten infrage kommen: Alkoxy mit 1 bis 4 Kohlenstoffatomen, unsubstituiertes oder ein- bis fünffach, gleich oder verschieden durch Halogen, Nitro, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen, Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil substituiertes Phenyl, für Cycloalkyl oder Cycloalkenyl mit 3 bis 6 Kohlenstoffatomen, $R^4$ und $R^5$ weiterhin für Alkenyl oder Alkinyl mit 3 bis 6 Kohlenstoffatomen, unsubstituiertes oder ein- bis fünffach, gleich oder verschieden substituiertes Phenyl, wobei als Phenylsubstituenten die oben bei $R^4$ und $R^5$ als Phenylsubstituenten genannten Substituenten infrage kommen oder für ein- bis dreifach, gleich oder verschieden substituiertes 3- bis 6-gliedriges Heterocyclyl mit 1 bis 3 gleichen oder verschiedenen Heteroatomen, wie Stickstoff, Sauerstoff und Schwefel, wobei folgende Substituenten infrage kommen: Halogen, Hydroxy, Alkyl oder Alkoxy mit 1 bis 4 Kohlenstoffatomen und Oxo; ferner $R^4$ und $R^5$ gemeinsam mit dem Stickstoffatom, an welches sie gebunden sind, für einen ein- bis dreifach, gleich oder verschieden substituierten oder unsubstituierten 5- oder 6-gliedrigen Heterocyclus, der 1 oder 2 weitere, gleiche oder verschiedene Heteroatome, wie Stickstoff, Sauerstoff oder Schwefel, enthalten kann, wobei als Substituenten Halogen, Hydroxy, Alkyl und Alkoxy mit 1 bis 4 Kohlenstoffatomen und Oxo infrage kommen,

$R^6$ für Wasserstoff, 1- bis 5-fach, gleich oder verschieden substituiertes oder unsubstituiertes, geradkettiges oder verzweigtes Alkyl mit 1 bis 6 Kohlenstoffatomen, wobei als Substituenten infrage kommen: Halogen, Alkoxy mit 1 bis 4 Kohlenstoffatomen, unsubstituiertes oder substituiertes Cycloalkyl mit 3 bis 6 Kohlenstoffatomen, ein- bis dreifach substituiertes oder unsubstituiertes Phenyl, wobei als Substituenten die oben bei $R^4$ und $R^5$ als Phenylsubstituenten genannten infrage kommen; $R^6$ ferner für Alkenyl oder Alkinyl mit 2 bis 6 Kohlenstoffatomen, Cycloalkyl mit 3 bis 6 Kohlenstoffatomen, ein- bis dreifach, gleich oder verschieden substituiertes oder unsubstituiertes Phenyl, wobei als Phenylsubstituenten die oben bei $R^4$ und $R^5$ als Phenylsubstituenten genannten Substituenten infrage kommen, unsubstituiertes oder ein bis dreifach, gleich oder verschieden substituiertes 3- bis 6-gliedriges Heterocyclyl mit 1 bis 3 gleichen oder verschiedenen Heteroatomen, wie Stickstoff, Sauerstoff und Schwefel, wobei als Substituenten Halogen, Hydroxy, Alkyl oder Alkoxy mit jeweils 1 bis 4 Kohlenstoffatomen oder Oxo infrage kommen und weiterhin für Alkoxycarbonyl mit 1 oder 2 Kohlenstoffatomen oder Phenylcarbonyl,

$R^7$ für unsubstituiertes oder ein- bis fünffach durch Chlor substituiertes Alkyl mit 1 bis 6 Kohlenstoffatomen oder für unsubstituiertes oder ein- bis dreifach, gleich oder verschieden substituiertes Phenyl steht, wobei als Phenylsubstituenten die oben bei $R^4$ und $R^5$ als Phenylsubstituenten genannten Substituenten infrage kommen.

Besonders bevorzugt sind Verbindungen der Formel (I), in welcher

$R^1$ für unsubstituiertes Alkyl mit 1 bis 8 Kohlenstoffatomen oder ein- bis dreifach, gleich oder verschieden substituiertes, geradkettiges oder verzweigtes Alkyl mit 1 bis 5 Kohlenstoffatomen steht, wobei folgende Substituenten infrage kommen: Fluor, Chlor, Brom, Iod, Cyano, -COOH, $-COOR^3$, $-CONR^4R^5$, $-OR^6$, $-SR^6$, Alkylcarbonyl mit 1 bis 7 Kohlenstoffatomen, unsubstituiertes oder ein- oder zweifach durch Halogen

substituiertes Phenylcarbonyl, unsubstituiertes oder ein- bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylmethoxy substituiertes Phenyl, weitere Alkylsubstituenten sind jeweils unsubstituiertes oder ein- bis dreifach durch Methyl oder Chlor substituiertes Cyclopropyl, Cyclopentyl, Cyclohexyl, Cyclopentenyl oder Cyclohexenyl und unsubstituiertes oder einfach bis dreifach, gleich oder verschieden durch Fluor, Chlor, Methyl, Methoxy oder Hydroxy substituierte Heterocyclen der Formeln

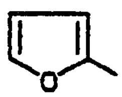 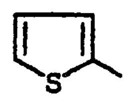 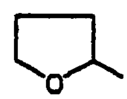 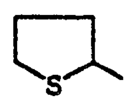 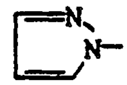

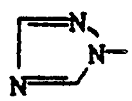 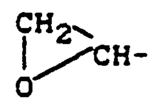 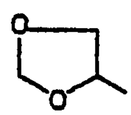 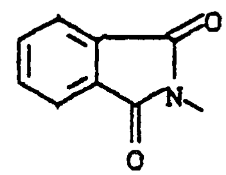

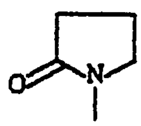 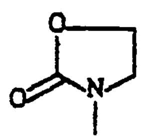 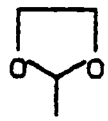 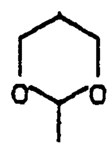 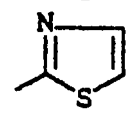 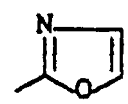

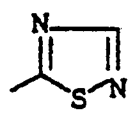 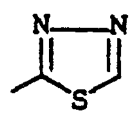 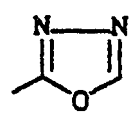 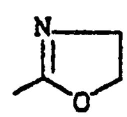 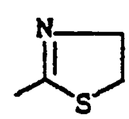 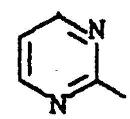

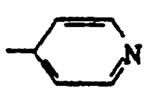 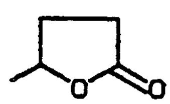 ,

$R^1$ ferner für ein- oder zweifach substituiertes Allyl oder Propargyl steht, wobei als Substituenten unsubstituiertes oder ein- bis dreifach, gleich oder verschieden durch Fluor, Chlor, Methyl, Methoxy substituiertes Phenyl genannt seien. $R^1$ weiterhin für jeweils unsubstituiertes oder ein- bis dreifach durch Chlor, Methyl oder Methoxy substituiertes Cyclopropyl, Cyclopentyl, Cyclohexyl, Cyclopentenyl oder Cyclohexenyl steht;

$R^2$ für Wasserstoff, für unsubstituiertes oder ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, -COOH, -$COOR^3$, -$CONR^4R^5$, -$OR^6$, -$SR^6$, Alkylcarbonyl mit 1 bis 7 Kohlenstoffatomen, unsubstituiertes oder ein- bis dreifach, gleich oder verschieden durch Halogen substituiertes Phenylcarbonyl, unsubstituiertes oder ein- bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylalkoxy mit 1 oder 2 Kohlenstoffatomen im Alkoxyteil substituiertes Phenyl; weitere Alkylsubstituenten sind jeweils unsubstituiertes oder ein- bis dreifach durch Methyl oder Chlor substituiertes Cyclopropyl, Cyclopentyl, Cyclohexyl, Cyclopentenyl oder Cyclohexenyl oder unsubstituierte oder einfach bis dreifach, gleich oder verschieden durch Fluor, Chlor, Methyl, Methoxy oder Hydroxy substituierte Heterocyclen der Formeln

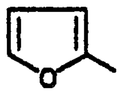 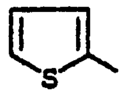 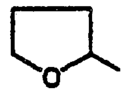 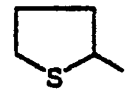 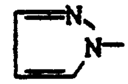

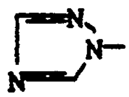 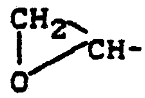 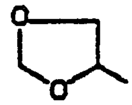 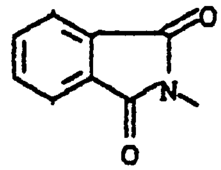

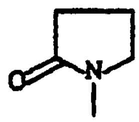 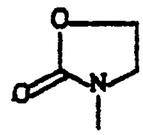 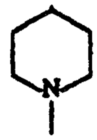 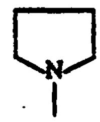 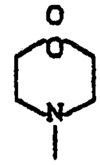 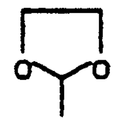

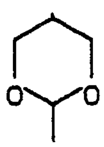 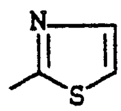 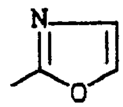 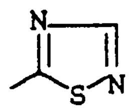 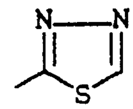

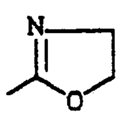 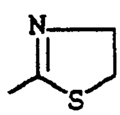 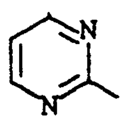 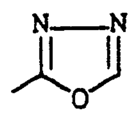

$R^2$ steht ferner für ein- oder zweifach, gleich oder verschieden substituiertes Allyl oder Propargyl, wobei als Substituenten unsubstituiertes oder einbis dreifach, gleich oder verschieden durch Fluor, Chlor, Methyl oder Methoxy substituiertes Phenyl genannt seien; weiterhin steht $R^2$ für jeweils unsubstituiertes oder ein- bis dreifach durch Methyl oder Methoxy substituiertes Cyclopropyl, Cyclopentyl, Cyclohexyl, Cyclopentenyl oder Cyclohexenyl,

$R^2$ steht ferner für unsubstituiertes oder ein- bis dreifach, gleich oder verschieden substituiertes Phenyl, wobei als Substituenten genannt seien: Fluor, Chlor, Nitro, Methyl, Methoxy, Trifluormethyl, Trifluormethoxy,

$R^2$ steht ferner für unsubstituiertes oder ein- bis dreifach, gleich oder verschieden durch Fluor, Chlor, Hydroxy, Oxo, Methyl oder Methoxy substituierte Heterocyclen der Formeln

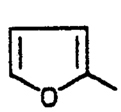 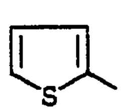 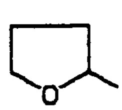 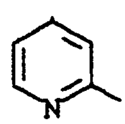 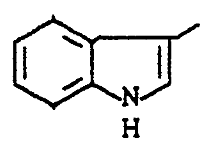

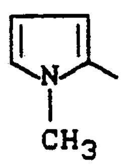 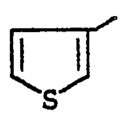 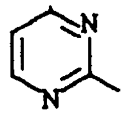 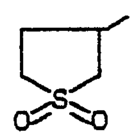

oder für einen der Reste $-CX-YR^3$, $-CXNR^4R^5$, $-COR^6$, $-SO_2-NR^4R^5$ oder $-SO_2R^7$ steht,

worin

X und Y gleich oder verschieden sind und für Sauerstoff oder Schwefel stehen,

$R^3$ für unsubstituiertes oder ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, unsubstituiertes oder einfach bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylalkoxy mit 1 oder 2 Kohlenstoffatomen im Alkylteil substituiertes Phenyl und jeweils unsubstituiertes oder ein- bis dreifach durch Methyl substituiertes Cyclopropyl, Cyclopentyl, Cyclohexyl, Cyclopentenyl, Cyclohexenyl; $R^3$ weiterhin für Allyl oder Propargyl steht,

$R^4$ und $R^5$ gleich oder verschieden sind und für Wasserstoff oder für unsubstituiertes oder gleich oder verschieden, ein- oder zweifach substituiertes Alkyl mit 1 bis 4 Kohlenstoffatomen stehen, wobei folgende Substituenten infrage kommen: Methoxy, Ethoxy, unsubstituiertes oder ein- bis dreifach, gleich oder verschieden durch Fluor, Chlor, Brom, Iod, Nitro, Methyl und Methoxy, Trifluormethyl, Trifluormethoxy, Amino, Methylamino, Dimethylamino, Phenoxy, Phenylmethoxy substituiertes Phenyl;

$R^4$ und $R^5$ weiterhin für Allyl, Propargyl, Cyclopropyl, Cyclopentyl, Cyclohexyl, Cyclopentenyl, Cyclohexenyl, unsubstituiertes oder ein- bis dreifach, gleich oder verschieden substituiertes Phenyl stehen, wobei als Phenylsubstituenten die oben bei $R^4$ und $R^5$ als Phenylsubstituenten genannten Substituenten infrage kommen, oder ferner für einfach bis dreifach, gleich oder verschieden durch Fluor, Chlor, Methyl, Methoxy, Hydroxy oder Oxo substituierte Heterocyclen der Formeln

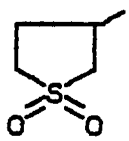
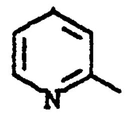
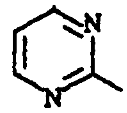
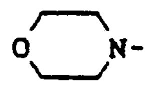
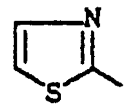
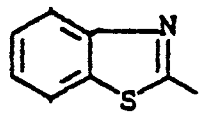
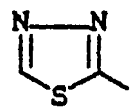
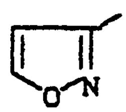
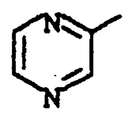
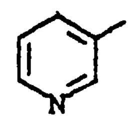
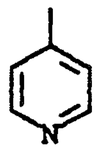
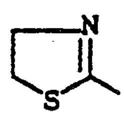 stehen,

oder

ferner $R^4$ und $R^5$ gemeinsam mit dem Stickstoffatom, an welches sie gebunden sind, für jeweils unsubstituiertes oder substituiertes Oxazolin, Pyrrolidin, Imidazolin, Piperazin, Morpholin, Thiomorpholin, 1,3-Oxazan oder 1,3-Diazan stehen, wobei als Substituenten Fluor, Chlor, Brom, Iod, Hydroxy, Methyl, Methoxy und Oxo infrage kommen,

$R^6$ für Wasserstoff, für unsubstituiertes oder ein- bis dreifach, gleich oder verschieden substituiertes geradkettiges oder verzweigtes Alkyl mit 1 bis 4 Kohlenstoffatomen steht, wobei als Substituenten infrage kommen: Fluor, Chlor, Brom, Iod, Methoxy, Cyclopropyl, Cyclopentyl, Cyclohexyl, ein- oder zweifach, gleich oder verschieden substituiertes oder unsubstituiertes Phenyl, wobei als Phenylsubstituenten die oben bei $R^4$ und $R^5$ als Phenylsubstituenten genannten infrage kommen; ferner für Allyl oder Propargyl, Cyclopropyl, Cyclopentyl, Cyclohexyl oder für ein- oder zweifach, gleich oder verschieden substituiertes oder unsubstituiertes Phenyl steht, wobei als Phenylsubstituenten die oben bei $R^4$ und $R^5$ als Phenylsubstituenten genannten Substituenten infrage kommen; weiterhin für unsubstituiertes oder 1- bis 3-fach, gleich oder verschieden durch Fluor, Chlor, Brom, Iod, Hydroxy, Oxo, Methyl, Methoxy substituierte Heterocyclen der Formeln

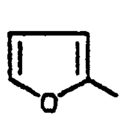
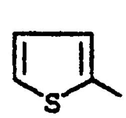
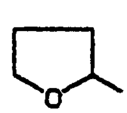
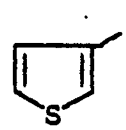
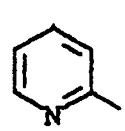
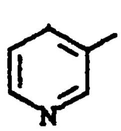
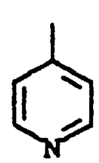
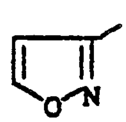 steht

und weiterhin für Methoxycarbonyl oder Ethoxycarbonyl oder Phenylcarbonyl steht,

$R^7$ für unsubstituiertes oder ein- bis dreifach durch Chlor substituiertes Alkyl mit 1 bis 4 Kohlenstoff atomen oder für unsubstituiertes oder ein- bis dreifach, gleich oder verschieden substituiertes Phenyl steht, wobei als Phenylsubstituenten die oben bei $R^4$ und $R^5$ als Phenylsubstituenten genannten Substituenten infrage kommen.

Ganz besonders bevorzugt sind Verbindungen der Formel (I), in welcher

$R^1$ für unsubstituiertes Alkyl mit 1 bis 8 Kohlenstoffatomen oder für ein- oder zweifach, gleich oder verschieden substituiertes Methyl, Ethyl, n- oder i-Propyl steht, wobei folgende Substituenten infrage kommen: Fluor, Cyano, -COOH, $-COOR^3$, $-CONR^4R^5$, $-OR^6$, $-SR^6$, Alkylcarbonyl mit 1 bis 5 Kohlenstoffatomen, unsubstituiertes oder ein- oder zweifach, gleich oder verschieden durch Fluor, Chlor, Brom substituiertes Phenylcarbonyl, unsubstituiertes oder ein- bis dreifach, gleich oder verschieden durch Fluor, Chlor, Nitro, Methyl, Methoxy, Trifluormethyl, Methylamino, Dimethylamino und Phenylmethoxy substituiertes Phenyl; weitere Alkylsubstituenten sind jeweils unsubstituiertes oder ein-oder zweifach durch Methyl oder Chlor substituiertes Cyclopropyl, Cyclohexyl oder Cyclohexenyl oder unsubstituiertes oder einfach bis dreifach durch Methyl substituierte Heterocyclen der Formeln

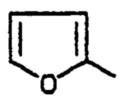 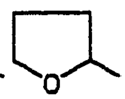 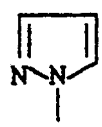 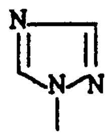 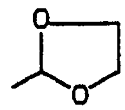 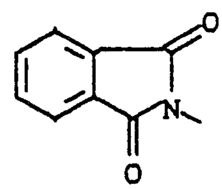 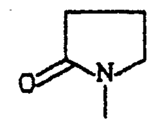

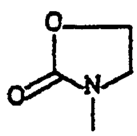 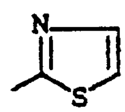 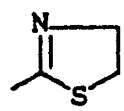 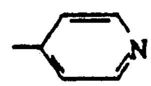 und 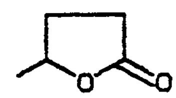

stehen,

$R^1$ ferner für durch Phenyl substituiertes Allyl oder Propargyl oder für jeweils unsubstituiertes oder ein- oder zweifach durch Methyl oder Chlor substituiertes Cyclohexyl, Cyclopropyl oder Cyclohexenyl steht,

$R^2$ für Wasserstoff, für unsubstituiertes oder ein- bis dreifach, gleich oder verschieden substituiertes Methyl oder Ethyl steht, wobei folgende Substituenten infrage kommen: Fluor, Chlor, Cyano, -COOH, $-COOR^3$, $-CONR^4R^5$, $-OR^6$, $-SR^6$, Alkylcarbonyl mit 1 bis 5 Kohlenstoffatomen, unsubstituiertes oder einoder zweifach, gleich oder verschieden durch Fluor, Chlor oder Brom substituiertes Phenylcarbonyl, Phenyl, Cyclopropyl, 1,3-Dioxolan-2-yl oder 1,2,4-Triazol-1-yl; $R^2$ weiterhin für Allyl, Propargyl, für Cyclohexyl, unsubstituiertes oder ein- oder zweifach, gleich oder verschieden durch Chlor, Methyl oder Methoxy substituiertes Phenyl oder für einen der Reste $-CX-YR^3$, $-CXNR^4R^5$, $-COR^6$, $-SO_2-NR^4R^5$, $-SO_2R^7$ oder

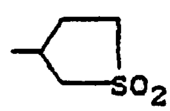

steht,

worin

X und Y gleich oder verschieden sind und für Sauerstoff oder Schwefel stehen,

$R^3$ für Methyl, Ethyl, n- oder i-Propyl, n-, i-, s-oder t-Butyl steht,

$R^4$ für Wasserstoff, Methyl, Ethyl, n- oder i-Propyl, n-, i-, s- oder t-Butyl oder Phenyl steht,

$R^5$ für Wasserstoff, Methyl, Ethyl, n- oder i-Propyl, n-, i-, s- oder t-Butyl oder Phenyl steht,

$R^6$ für Wasserstoff, unsubstituiertes oder ein- bis dreifach durch Fluor, Chlor, Methoxy oder Phenyl substituiertes Methyl, Ethyl, n- oder i-Propyl oder n-, i-, s- oder t-Butyl steht; $R^6$ ferner für Phenyl, Methoxycarbonyl, Ethoxycarbonyl oder Phenylcarbonyl steht und

$R^7$ für Methyl, Ethyl, n- oder i-Propyl oder n-, i-, s- oder t-Butyl oder für unsubstituiertes oder einoder zweifach durch Methyl substituiertes Phenyl steht.

Weiterhin sind ganz besonders bevorzugt Verbindungen der Formel (I), in welcher

$R^1$ für unsubstituiertes, geradkettiges oder verzweigtes Alkyl mit 1 bis 6 Kohlenstoffatomen, für einoder zweifach, gleich oder verschieden substituiertes Methyl, Ethyl, n- oder i-Propyl steht, wobei als Substituenten genannt seien: Fluor, Cyano, -COOH, $-COOR^3$, $-CONR^4R^5$, $-OR^6$, $-SR^6$, Alkylcarbonyl mit 1 bis 5 Kohlenstoffatomen im Alkylteil, unsubstituiertes oder ein- oder zweifach, gleich oder verschieden durch Brom substituiertes Phenylcarbonyl, unsubstituiertes oder ein- bis dreifach, gleich oder verschieden durch

Chlor, Methyl, Methoxy, Nitro, Dimethylamino, Benzyloxy und Trifluormethyl substituiertes Phenyl, unsubstituiertes Cyclopropyl, Cyclohexyl oder Cyclohexenyl, ein- oder zweifach, gleich oder verschieden durch Chlor und Methyl substituiertes Cyclopropyl, unsubstituierte oder ein- bis dreifach durch Methyl, substituierte Heterocyclen der Formel

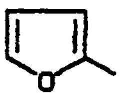 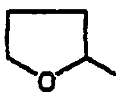 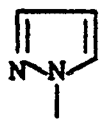 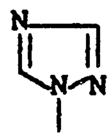 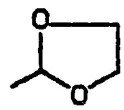 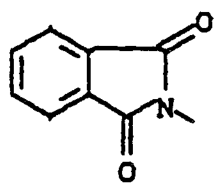 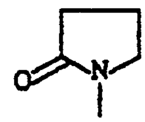

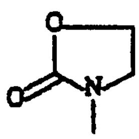 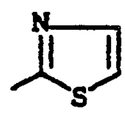 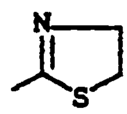 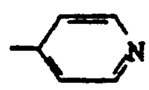 und 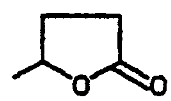

$R^1$ steht weiterhin für Allyl, Phenylallyl, Propargyl, Cyclohexyl oder Cyclohexenyl,
$R^2$ steht für Wasserstoff, geradkettiges oder verzweigtes Alkyl mit 1 bis 4 Kohlenstoffatomen, Alkylcarbonyl mit 1 bis 3 Kohlenstoffatomen im geradkettigen oder verzweigten Alkylteil, Alkylaminocarbonyl mit 1 oder 2 Kohlenstoffatomen im Alkylteil, für einfach durch Alkoxy oder Alkoxycarbonyl mit 1 bis 3 Kohlenstoffatomen im Alkoxyteil, Cyano, Hydroxy, Aminocarbonyl, Phenyl, -COOH, Phenylcarbonyl, Cyclopropyl, Triazolyl oder Dioxolanyl substituiertes geradkettiges oder verzweigtes Alkyl mit 1 bis 3 Kohlenstoffatomen, Halogenalkyl mit 1 bis 3 Kohlenstoffatomen und 1 bis 5 gleichen oder verschiedenen Halogenatomen, wie Fluor und Chlor, Phenylsulfonyl, Phenyl oder ein- bis dreifach, gleich oder verschieden durch Chlor und Methyl substituiertes Phenyl, Alkylthiocarbonyl mit 1 bis 3 Kohlenstoffatomen im geradkettigen oder verzweigten Alkylthioteil, Alkenyl oder Alkinyl mit 3 oder 4 Kohlenstoffatomen und
$R^3$ - $R^6$ die gegebenen Bedeutungen haben.

$R^6$ in der Definition von $OR^6$ in $R^1$ und von $COR^6$ in $R^2$ kann in den beiden Fällen gleich oder verschieden sein.

Alle aliphatischen Reste als solche oder in Zusammensetzungen können geradkettig oder verzweigt sein, auch wenn es nicht ausdrücklich vermerkt ist.

Im einzelnen seien außer den bei den Herstellungsbeispielen genannten Verbindungen die folgenden 3-Amino-2-pyrazolin-5-on-Derivate der allgemeinen Formel (I)

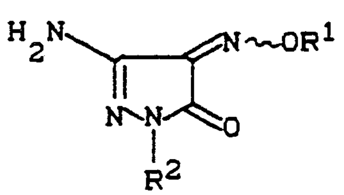

(I)

genannt:

Tabelle 1:

| $R^1$ | $R^2$ | $R^1$ | $R^2$ |
|---|---|---|---|
| 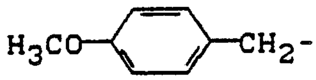 | H | 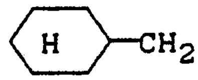 | H |
| 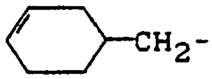 | H | 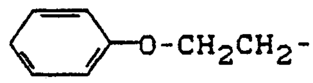 | H |
| 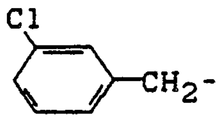 | H | 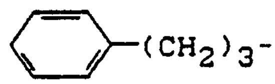 | H |
| 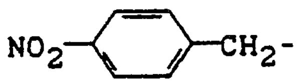 | H | $HC{\equiv}C-CH_2-$ | H |
| 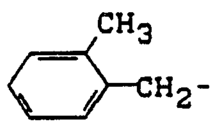 | H | $CH_3-(CH_2)_7-$ | H |
| 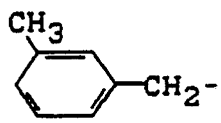 | H | $C_4H_9O-CH_2CH_2-$ | H |
| 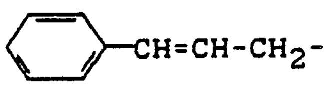 | H | $C_2H_5S-CH_2CH_2-$ | H |
| | | $t-C_4H_9-CO-CH_2-$ | H |
| | | 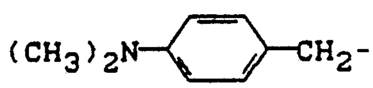 | H |
| | | 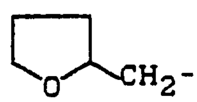 | H |

Tabelle 1: - Fortsetzung

| $R^1$ | $R^2$ |
|---|---|
| 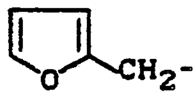 | H |
| 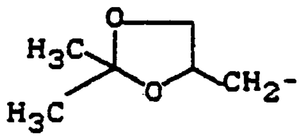 | H |
| 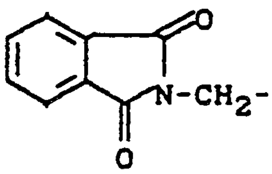 | H |
| 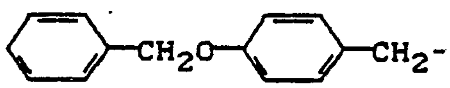 | H |
| 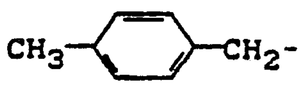 | $CH_3$ |
| 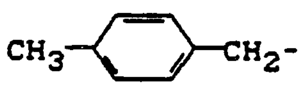 | $-CH_2-CO-NH_2$ |
| 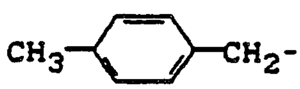 | $-CH_2-CN$ |
| 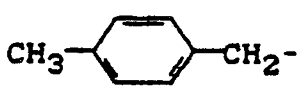 | $-CH_2-CH_2-OH$ |
| 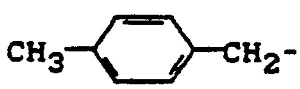 | 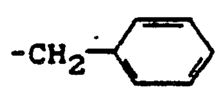 |
| 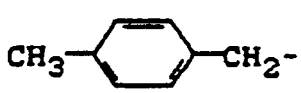 | $-CH_2-CH=CH_2$ |

Tabelle 1: - Fortsetzung

| $R^1$ | $R^2$ |
|---|---|
| 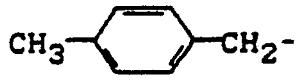 | $-COOCH_3$ |
| 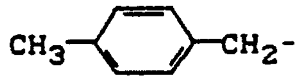 | $-SO_2-N(CH_3)_2$ |
| 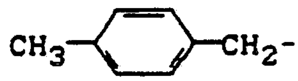 | $-CO-SC_2H_5$ |
| 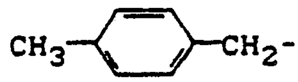 | $-CH_2-COOH$ |
| 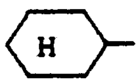 | H |
| $-CH_2-S-CH_3$ | H |
| 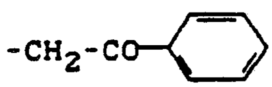 | H |
| 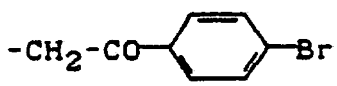 | H |
| 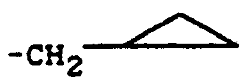 | H |
| 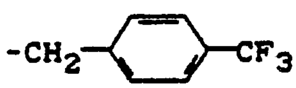 | H |
| $-CH_2-CH_2-N(CH_3)_2$ | H |

<u>Tabelle 1</u>: - Fortsetzung

| $R^1$ | $R^2$ |
|---|---|
| 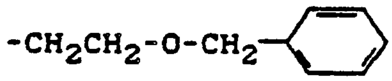 | H |
| 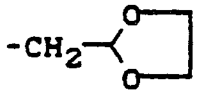 | H |
| 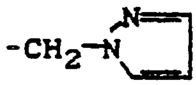 | H |
| 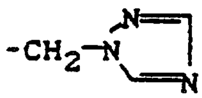 | H |
| 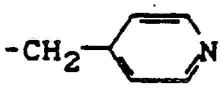 | H |
| 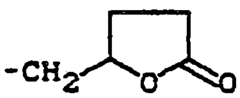 | H |
| 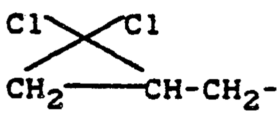 | H |
| 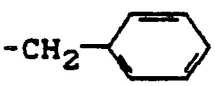 | $-C_2H_5$ |
| 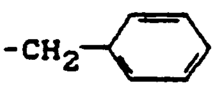 | $-CH_2-CCl_3$ |
| 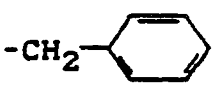 | $-CH_2-CF_3$ |
| 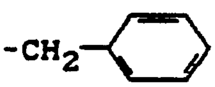 | 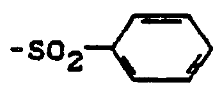 |
| 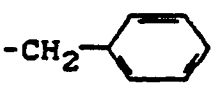 | $-CH_2-O-CH_3$ |
| 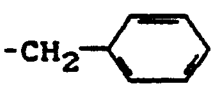 | 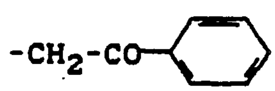 |

Tabelle 1: - Fortsetzung

| $R^1$ | $R^2$ |
|---|---|
| 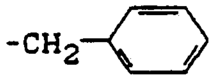 | $-CH_2-CO-CH_3$ |
| 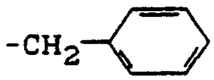 | 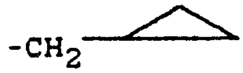 |
| 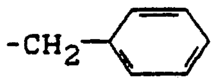 | 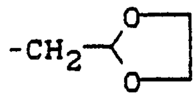 |
| 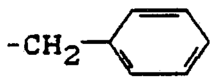 | 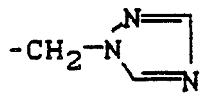 |
| 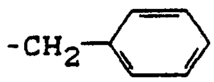 | $-CH_2-C\equiv CH$ |
| 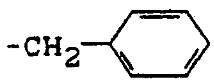 | 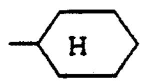 |
| 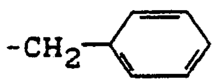 | 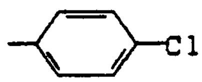 |
| 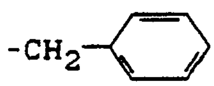 | 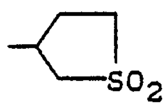 |
| 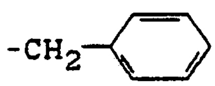 | 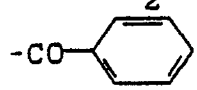 |
| $-CHF_2$ | H |
| $-CH_2-CN$ | H |
| $-CH_2-COOH$ | H |
| $-CH_2-CO-NH_2$ | H |
| $-CH_2-CO-N(C_2H_5)_2$ | H |
| 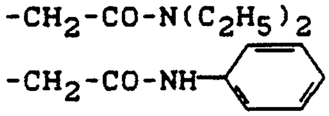 | H |
| $-CH_2-O-CH_3$ | H |

Verwendet man als Ausgangsstoffe beispielsweise (E)-2-Benzyloximino-2-cyan-acetylchlorid und Methylhydrazin, Triethylamin als Base und 4-Dimethylaminopyridin als Katalysator, so läßt sich der Reaktionsablauf des erfindungsgemäßen Verfahrens (a) durch das folgende Formelschema darstellen:

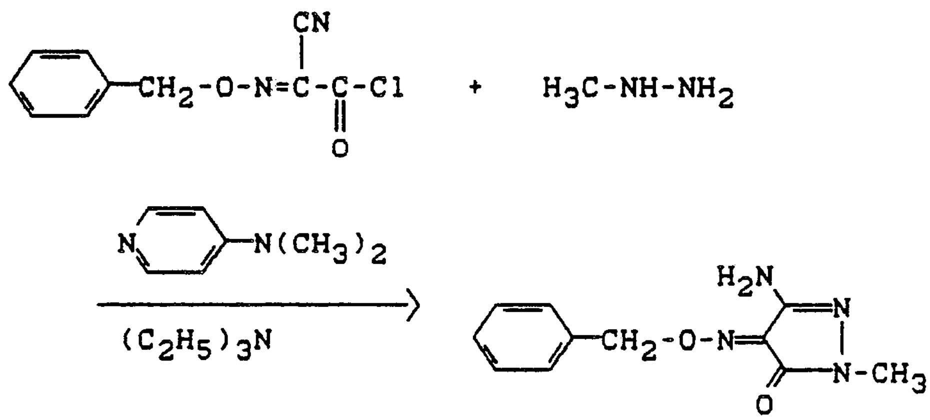


Verwendet man als Ausgangsstoffe beispielsweise (E)-3-Amino-4-benzyloximino-2-pyrazolin-5-on und Methylisocyanat und Triethylamin als Base, so läßt sich der Reaktionsablauf des erfindungsgemäßen Verfahrens (b3) durch das folgende Formelschema darstellen:

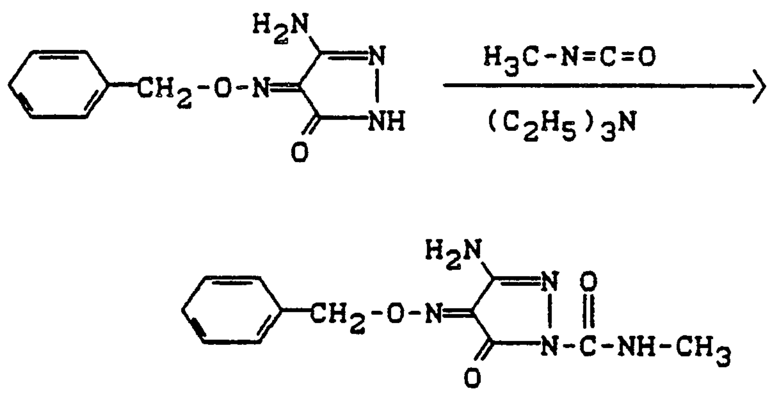


Verwendet man als Ausgangsstoffe beispielsweise das Natriumsalz des (E)-3-Amino-4-hydroximino-2-pyrazolin-5-ons und Bromessigsäureethylester, so läßt sich der Reaktionsablauf des erfindungsgemäßen Verfahrens (c) durch das folgende Formelschema darstellen:

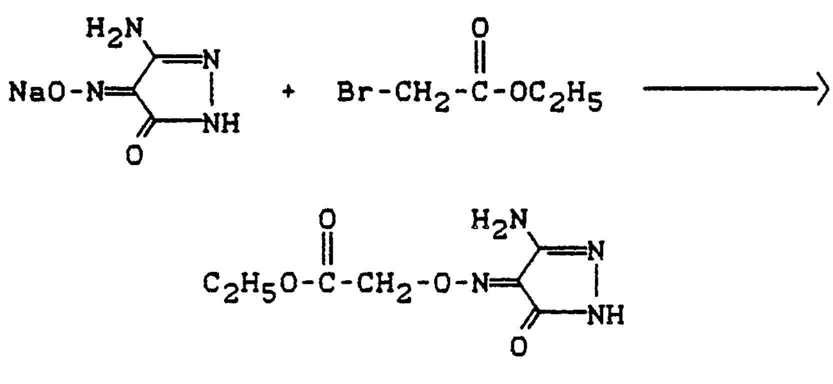

Die zur Durchführung des erfindungsgemäßen Verfahrens (a) als Ausgangsstoffe benötigten 2-Cyan-2-oximinoessigsäure-Derivate sind durch die Formel (II) allgemein definiert. In dieser Formel (II) steht $R^1$ vorzugsweise für diejenige Bedeutung, die schon bei der Beschreibung der erfindungsgemäßen Stoffe der Formel (I) für diesen Substituenten als bevorzugt genannt wurde. $R^8$ steht vorzugsweise für Methoxy, Ethoxy oder Chlor.

Die Verbindungen der Formel (II) sind bekannt (vgl. hierzu z.B. DE-OS 37 19 226) bzw. können nach den dort beschriebenen Verfahren erhalten werden.

Die außerdem für die Durchführung des erfindungsgemäßen Verfahrens (a) als Ausgangsstoffe zu

verwendenden Hydrazine sind durch die Formel (III) allgemein definiert.

In dieser Formel (III) hat $R^2$ die Bedeutungen, die bereits im Zusammenhang mit der Beschreibung der erfindungsgemäßen Stoffe der Formel (I) als bevorzugt für diesen Substituenten genannt wurden.

Die Hydrazine der Formel (III) sind allgemein bekannte Verbindungen der organischen Chemie.

Als Verdünnungsmittel zur Durchführung des erfindungsgemäßen Verfahrens (a) kommen organische Lösungsmittel oder wäßrige Systeme infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan Dichlormethan, Chloroform, Tetrachlorkohlenstoff; Ether, wie Diethylether, Dioxan, Tetrahydrofuran oder Ethylenglykoldimethyl- oder -diethylether; Alkohole, wie Methanol oder Ethanol; Nitrile, wie Acetonitril oder Propionitril; Amide, wie Dimethylformamid, Dimethylacetamid, N-Methylformanilid, N-Methylpyrrolidon oder Hexamethylphosphorsäuretriamid oder auch Wasser oder wäßrigorganische Zweiphasen-Gemische, wie Dichlormethan-Wasser oder Toluol-Wasser.

Das erfindungsgemäße Verfahren (a) wird gegebenenfalls in Gegenwart eines Säurebindemittels durchgeführt.

Als solche kommen alle üblichen anorganischen oder organischen Basen infrage. Hierzu gehören beispielsweise Alkalimetallcarbonate, wie Natriumcarbonat, Kaliumcarbonat oder Natriumhydrogencarbonat sowie tertiäre Amine, wie Triethylamin, N,N-Dimethylanilin und Pyridin.

Das erfindungsgemäße Verfahren (a) wird gegebenenfalls in Gegenwart eines Katalysators durchgeführt. Beispielsweise genannt seien tertiäre Amine, wie N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20° C und +160° C, vorzugsweise bei Temperaturen zwischen 0° C und +120° C.

Bei der Durchführung des erfindungsgemäßen Verfahrens (a) setzt man pro Mol 2-Cyan-2-oximinoessigsäure-Derivat der Formel (II) im allgemeinen 1,0 bis 3,0 Mol, vorzugsweise 1,0 bis 2,0 Mol, Hydrazin der Formel (III), gegebenenfalls 1,0 bis 3,0 Mol, vorzugsweise 1,0 bis 2,0 Mol, einer Base und gegebenenfalls 0,001 bis 1,0 Mol, vorzugsweise 0,01 bis 0,2 Mol eines Katalysators ein.

Die Reaktionsdurchführung, Aufarbeitung und Isolierung der Reaktionsprodukte der Formel (I) erfolgt in allgemein üblicher Weise.

Die zur Durchführung der erfindungsgemäßen Verfahrensvarianten (b) als Ausgangsstoffe benötigten 4-Oximino-2-pyrazolin-5-on-Derivate sind durch die Formel (Ia) all gemein definiert. In dieser Formel (Ia) steht $R^1$ vorzugsweise für diejenigen Bedeutungen, die schon bei der Beschreibung der erfindungsgemäßen Stoffe der Formel (I) für diesen Substituenten als bevorzugt genannt wurden.

Die Verbindungen der Formel (Ia) sind erfindungsgemäße Verbindungen und erhältlich nach Verfahren (a) und (c). Die zur Durchführung des erfindungsgemäßen Verfahrens (b1) weiterhin als Ausgangsstoffe benötigten Alkylierungsmittel sind durch die Formel (IV) allgemein definiert. In dieser Formel (IV) steht $R^{2-1}$ für die unter der Formel (IV) genannten Reste und die entsprechenden Teile der Definitionen, die schon bei der Beschreibung der erfindungsgemäßen Stoffe der Formel (I) als bevorzugt und besonders bevorzugt für diese Substituenten genannt wurden. A steht vorzugsweise für Halogen, insbesondere Chlor, Brom oder Iod, oder für unsubstituiertes oder substituiertes Alkylsulfonyloxy, Alkoxysulfonyloxy oder Arylsulfonyloxy, wie beispielsweise Methansulfonyloxy, Methoxysulfonyloxy, Trifluormethansulfonyloxy oder p-Toluolsulfonyloxy.

Die Alkylierungsmittel der Formel (IV) sind allgemein bekannte Verbindungen der organischen Chemie.

Die außerdem zur Durchführung der erfindungsgemäßen Verfahrensvarianten (b2) und (b3) als Ausgangsstoffe zu verwendenden Acylierungsreagenzien sind durch die Formeln (Va) und (Vb) allgemein definiert. In der Formel (Va) steht Z vorzugsweise für eine Abgangsgruppe. Hierzu gehören vorzugsweise Chlor, Brom, Alkoxy und Alkylthio mit jeweils 1 bis 4 Kohlenstoffatomen, Carboxymethoxy, Carboxymethylthio, die Gruppierungen $-O-CO-R^{2-2}$, $-O-CO-OR^{2-1}$, $-OR^{2-1}$ und $-SR^{2-1}$. Dabei haben $R^{2-2}$ und $R^{2-1}$ vorzugsweise die Bedeutungen, die bereits im Zusammenhang mit den Formeln (Va) und (IV) und mit der Beschreibung der erfindungsgemäßen Stoffe der Formel (I) für die entsprechenden Teile der Definitionen der Substituenten vorzugsweise genannt wurden.

In der Formel (Vb) haben X und $R^{2-3}$ vorzugsweise die Bedeutungen, die bereits im Zusammenhang mit der Formel (Vb) und mit der Beschreibung der erfindungsgemäßen Stoffe der Formel (I) für die entsprechenden Teile der Definitionen der Substituenten vorzugsweise genannt wurden.

Die Acylierungsreagenzien der Formeln (Va) und (Vb), d.h. Carbonsäurehalogenide, Carbonsäureanhydride, Halogenameisensäurester und -thiolester, Trithiocarbonate, Pyrocarbonate, Carbamidsäurehalogenide, Carbamate, Thiolcarbamate, Dithiocarbamate, Isocyanate oder Isothiocyanate sind allgemein bekannte Verbindungen der organischen Chemie und nach allgemein üblichen Methoden erhältlich.

Als Verdünnungsmittel kommen für das erfindungsgemäße Verfahren (b) inerte aprotische organische Lösungsmittel infrage. Hierzu gehören vorzugsweise Ether, wie Tetrahydrofuran oder 1,2-Dimethoxyethan, Nitrile, wie Acetonitril, Amide, wie Dimethylformamid oder N-Methylpyrrolidon, Sulfoxide, wie Dimethylsulfoxid, Ester, wie Essigsäureethylester oder wäßrig-organische Zweiphasen-Gemische, wie Wasser-Toluol oder Wasser-Dichlormethan. Die erfindungsgemäßen Verfahrensvarianten (b) werden gegebenenfalls in Gegenwart eines Säurebindemittels durchgeführt.

Als solche kommen alle üblichen anorganischen oder organischen Basen infage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid, Alkalimetallcarbonate, wie Natriumcarbonat, Kaliumcarbonat oder Natriumhydrogencarbonat, tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU) sowie metallorganische Verbindungen, wie Butyllithium und Lithiumdiisopropylamid.

Die erfindungsgemäßen Verfahrensvarianten (b) werden gegebenenfalls in Gegenwart eines Katalysators durchgeführt. Beispielsweise genannt seien tertiäre Amine, wie 4-Dimethylaminopyridin, 1,8-Diazabicyclo[5,4,0]-undec-7-en (DBU) oder 1,4-Diazabicyclo[2,2,2]-octan (DABCO); ferner Imidazol und Dimethylformamid.

Führt man die Umsetzungen in einem organisch-wässrigen Zweiphasensystem durch, kann man gegebenenfalls in Gegenwart von 0,1 bis 1 Mol eines geeigneten Phasentransferkatalysators, wie beispielsweise einer quartären Ammonium- oder Phosphoniumverbindung arbeiten. Beispielhaft seien Triethylbenzylammoniumchlorid und Benzyldodecyl-dimethyl-ammoniumchlorid genannt.

Bei der Durchführung der erfindungsgemäßen Verfahrensvarianten (b1) - (b3) setzt man pro Mol 4-Alkoximino-2-pyrazolin-5-on-Derivat der Formel (Ia) im allgemeinen 1,0 bis 1,5 Mol, vorzugsweise 1,0 bis 1,1 Mol an Alkylierungsmitteln der Formel (IV) bzw. 1,0 bis 1,5 Mol, vorzugsweise 1,0 bis 1,1 Mol an Acylierungsmitteln der Formeln (Va) bzw. (Vb), gegebenenfalls 1,0 bis 3,0 Mol, vorzugsweise 1,0 bis 1,5 Mol einer Base und gegebenenfalls 0,001 bis 1,0 Mol, vorzugsweise 0,01 bis 0,2 Mol eines Katalysators ein.

Die Reaktionstemperaturen können bei der Durchführung der erfindungsgemäßen Verfahren (b) in einem größeren Bereich variiert werden. Im allgemeinen arbeitet man zwischen $-20^\circ$ C und $+120^\circ$ C, vorzugsweise zwischen $0^\circ$ C und $+40^\circ$ C.

Die Aufarbeitung und Isolierung der Reaktionsprodukte der Formel (I) erfolgt nach üblichen Methoden.

Die zur Durchführung des erfindungsgemäßen Verfahrens (c) als Ausgangsstoffe benötigten 4-Oximino-2-pyrazolin-5-on-Derivate sind durch die Formel (VI) allgemein definiert. In dieser Formel (VI) steht M vorzugsweise für Wasserstoff oder für ein Natrium- oder Kaliumkation.

Die 4-Oximino-2-pyrazolin-5-on-Derivate der Formel (VI) sind noch nicht bekannt. Sie können jedoch in bekannter Art und Weise entsprechend Verfahrensvariante (a) hergestellt werden, indem man z.B. Alkalimetallsalze der 2-Cyan-2-oximino-essigsäure-Derivate der Formel (IIa)

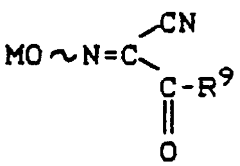

(IIa)

in welcher

M für ein Lithium-, Natrium- oder Kaliumkation steht und

$R^9$ für Methyl oder Ethyl steht,

mit Hydrazinhydrat der Formel (IIIa)

$H_2N-NH_2 \times H_2O$ (IIIa)

gegebenenfalls in Gegenwart eines Verdünnungsmittels, gegebenenfalls in Gegenwart einer Base und gegebenenfalls in Gegenwart eines Katalysators umsetzt.

Die Verbindungen der Formel (IIa) sind bekannt (vgl. hierzu Liebigs. Ann. Chem. 1981, 1561).

Hydrazinhydrat der Formel (IIIa) ist eine allgemein bekannte Verbindung der organischen Chemie.

Die zur Durchführung des erfindungsgemäßen Verfahrens (c) weiterhin als Ausgangsstoffe benötigten Alkylierungsmittel sind durch die Formel (VII) allgemein definiert. In dieser Formel (VII) steht $R^1$ vorzugsweise für diejenigen Reste, die bereits bei der Beschreibung der erfindungsgemäßen Stoffe der Formel (I) als bevorzugt für den Substituenten $R^1$ genannt wurden.

A steht vorzugsweise für diejenigen Abgangsgruppen, die bereits bei der Beschreibung der Alkylierungsmittel der Formel (IV) als bevorzugt für den Substituenten A genannt wurden.

Die Alkylierungsmittel der Formel (VII) sind ebenfalls allgemein bekannte Verbindungen der organischen Chemie.

Als Verdünnungsmittel zur Durchführung des erfindungsgemäßen Verfahrens (c) kommen organische Lösungsmittel oder wäßrige Systeme infrage.

Hierzu gehören insbesondere aromatische Kohlenwasserstoffe, wie Benzol, Toluol, Xylol; Ether, wie Tetrahydrofuran, Dioxan, Dimethoxyethan; Ketone, wie Aceton oder Methylisobutylketon; Nitrile, wie Acetonitril; Amide, wie Dimethylformamid oder N-Methylpyrrolidon; Ester, wie Essigester; Sulfoxide, wie Dimethylsulfoxid; Alkohole, wie Methanol oder Ethanol sowie Wasser, Mischungen der Lösungsmittel mit Wasser oder Gemische von mehreren Lösungsmitteln.

Führt man die Umsetzung in einem organisch-wäßrigen Zweiphasensystem durch, kann man gegebenenfalls in Gegenwart von 0,1 bis 1 Mol eines geeigneten Phasentransferkatalysators, wie beispielsweise einer quartären Ammonium- oder Phosphoniumverbindung arbeiten. Beispielhaft seien Triethylbenzylammoniumchlorid und Benzyldodecyl-dimethylammoniumchlorid genannt.

Die Reaktionstemperaturen können bei der Durchführung des erfindungsgemäßen Verfahrens (c) in einem größeren Bereich variiert werden. Im allgemeinen arbeitet man bei Temperaturen zwischen -20° C und +160° C, vorzugsweise bei Temperaturen zwischen 20° C und +100° C.

Bei der Durchführung des erfindungsgemäßen Verfahrens (c) setzt man die Reaktionsteilnehmer im allgemeinen vorzugsweise im äquimolaren Verhältnis ein. Unter bestimmten Bedingungen kann es jedoch vorteilhaft sein, einen Überschuß der einen oder anderen Komponente einzusetzen.

Die Aufarbeitung und Isolierung der Reaktionsprodukte der Formel (I) erfolgt nach üblichen Methoden.

Die erfindungsgemäßen Wirkstoffe weisen eine starke biologische Wirkung auf und können zur Bekämpfung von unerwünschten Schädlingen praktisch eingesetzt werden. Die Wirkstoffe sind z.B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;

Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindunsgemäßen Wirkstoffe mit besonders gutem Erfolg protektiv zur Bekämpfung von Pyricularia-Arten bei Reis eingesetzt werden.

Darüber hinaus zeigen einige der erfindungsgemäßen Wirkstoffe auch eine gute Wirkung gegen Oomyceten.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt-und Warmnebel-Formulierungen.

Diese Formulierungen werden in bekannter Weise herge stellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit so wie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulie rungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und Verwendung der erfindungsgemäßen Wirkstoffe geht aus den folgenden Beispielen hervor.

## Herstellungsbeispiele

### Beispiel 1

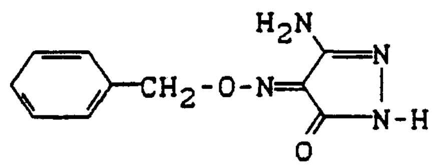

(Verfahren a)

Zu einer Lösung von 150,2 g (0,5 Mol) 78 %igem (E)-2-Benzyloximino-2-cyan-essigsäureethylester in 500 ml Ethanol tropft man bei Raumtemperatur 50 g (1 Mol) Hydrazinhydrat und rührt 15 Stunden bei Raumtemperatur. Der dunkelrotbraune Niederschlag wird abgesaugt, mit Ethanol gewaschen und unter vermindertem Druck bei 50° C getrocknet.

Man erhält 71,6 g (60 % der Theorie) (E)-3-Amino-4-benzyloximino-2-pyrazolin-5-on mit einem Gehalt von 92 % (HPLC = High pressure liquid chromatography) und einem Schmelzpunkt von 198-201° C (Zers.).

### Herstellung des Ausgangsmaterials

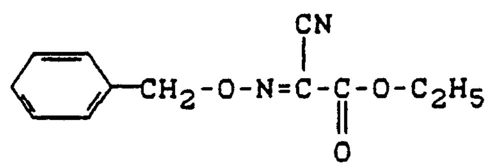

(II-1)

In eine Suspension von 328,2 g (2 Mol) des Natriumsalzes von (E)-2-Cyan-2-hydroximino-essigsäureethylester in 1500 ml Dimethylsulfoxid tropft man 253,1 g (2 Mol) Benzylchlorid und rührt 20 Stunden bei 60° C. Nach Zugabe von 1500 ml Wasser wird mit Essigester (3 x 500 ml) extrahiert, der Extrakt mit Wasser (3 x 500 ml) gewaschen, über Magnesiumsulfat getrocknet und unter vermindertem Druck eingedampft.

Man erhält 374,5 g (63 % der Theorie) eines rotbraunen Öls, mit einem Gehalt von 78% (E)-2-Benzyloximino-2-cyan-essigsäureethylester.

$^1$H-NMR *) : $\delta$ = 1,35 ppm (t, 3H); 4,35 (q, 2H); 5,45 (s, 2H); 7,35 (ar, 5H).

### Beispiel 2

*) Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

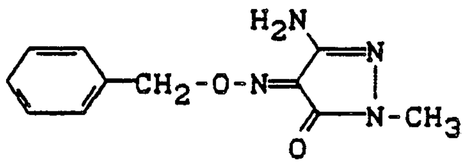

(Verfahren a)

In eine Lösung von 8,1 g (0,08 Mol) Triethylamin, 0,98 g (0,008 Mol) 4-Dimethylaminopyridin und 3,7 g (0,08 Mol) Methylhydrazin in 200 ml Methylenchlorid tropft man bei 0° C eine Lösung von 17,8 g (0,08 Mol) (E)-2-Benzyloximino-2-cyan-acetylchlorid in 100 ml Methylenchlorid und rührt 18 Stunden bei Raumtemperatur. Das Reaktionsgemisch wird mit 100 ml Wasser gewaschen, über Natriumsulfat getrocknet und unter vermindertem Druck eingedampft. Den Rückstand chromatographiert man an Kieselgel 60 mit Essigester.

Man erhält 8,9 g (48 % der Theorie) (E)-3-Amino-4-benzyloximino-1-methyl-2-pyrazolin-5-on als dunkelroten Feststoff vom Schmelzpunkt 105-108° C.

Herstellung des Ausgangsmaterials

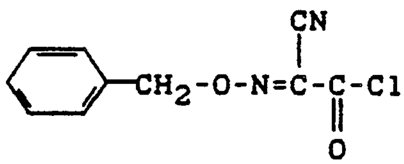

(II-2)

Zu einer Suspension von 48,5 g (0,2 Mol) des Kaliumsalzes der (E)-2-Benzyloximino-2-cyan-essigsäure in 200 ml absolutem Ether gibt man 2 Tropfen Dimethylformamid und tropft bei 0° C 126,9 g (1,0 Mol) Oxalylchlorid zu. Anschließend wird 2 Stunden bei 0° C gerührt. Das Reaktionsgemisch wird über Kieselgur filtriert, das Filtrat bei Raumtemperatur unter vermindertem Druck eingedampft und der Rückstand zweimal mit je 50 ml Methylenchlorid unter vermindertem Druck abgedampft.

Man erhält 40,2 g (90 % der Theorie) (E)-2-Benzyloximino-2-cyan-acetylchlorid, das sofort weiter umgesetzt wird.

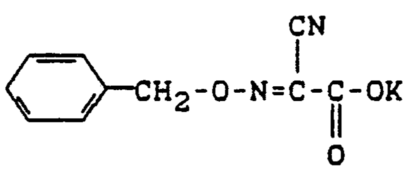

Zu einer Lösung von 59,5 g (0,2 Mol) 78%igem (E)-2-Benzyloximino-2-cyan-essigsäureethylester in 50 ml Ethanol tropft man bei 20 bis 30° C eine Lösung von 12,5 g (0,22 Mol) Kaliumhydroxid in 60 ml Ethanol und rührt 1 Stunde bei Raumtemperatur. Nach Verdünnen mit 100 ml Ether wird der Niederschlag abgesaugt, mit 100 ml Ether gewaschen und unter vermindertem Druck bei Raumtemperatur getrocknet.

Man erhält 41,2 g (85 % der Theorie) farbloses Kaliumsalz der (E)-2-Benzyloximino-2-cyan-essigsäure, das sich bei 166° C heftig zersetzt.

Beispiel 3

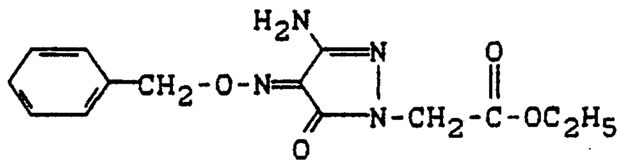

(Verfahren b2)

Zu einer Suspension von 23,7 g (0,1 Mol) 92 %igem (E)-3-Amino-4-benzyloximino-2-pyrazolin-5-on in 400 ml absolutem Tetrahydrofuran gibt man bei 20° C unter Rühren 3,2 g (0,01 Mol) Tetrabutylammoniumbromid und 12,2 g (0,105 Mol) 97%iges Kalium-tert-butylat. Sobald die Mischung homogen geworden ist, tropft man eine Lösung von 18,4 g (0,11 Mol) Bromessigsäureethylester in 50 ml absolutem Tetrahydrofuran zu und rührt 15 Stunden bei Raumtemperatur. Man gießt das Reaktionsgemisch in 1 l Wasser, extrahiert mit Essigester (3 x 500 ml), wäscht den Extrakt mit Wasser (3 x 500 ml), trocknet und dampft unter vermindertem Druck ein.

Man erhält 28,6 g (94 % der Theorie) rotbraunes (E)-3-Amino-4-benzyloximino-1-ethoxycarbonylmethyl-2-pyrazolin-5-on vom Schmelzpunkt 102-108° C.

Beispiel 4

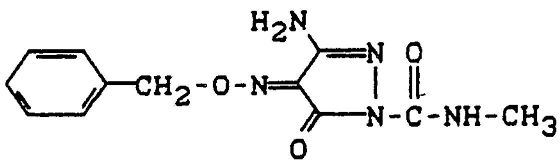

(Verfahren b3)

Man versetzt eine Suspension von 16,6 g (0,07 Mol) (E)-3-Amino-4-benzyloximino-2-pyrazolin-5-on in 130 ml Dioxan mit 3 Tropfen Triethylamin, tropft 4,0 g (0,07 Mol) Methylisocyanat zu und rührt 15 Stunden bei 70° C. Nach Abkühlung wird der Niederschlag abfiltriert, mit Dioxan gewaschen und unter vermindertem Druck getrocknet.

Man erhält 10,9 g (57 % der Theorie) rotbraunes (E)-3-Amino-4-benzyloximino-1-methylcarbamoyl-2-pyrazolin-5-on vom Schmelzpunkt 195-203° C.

Beispiel 5

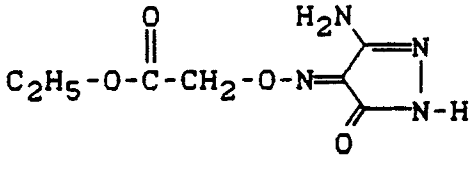

(Verfahren c)

15,0 g (0,1 Mol) des Natriumsalzes des (E)-3-Amino-4-hydroximino-2-pyrazolin-5-ons werden mit 17,0 g (0,1 Mol) 98 %igem Bromessigsäureethylester 6 Stunden bei 80° C gerührt. Das Reaktionsgemisch wird eingedampft und der Rückstand an Kieselgel 60 mit Essigester chromatographiert.

Man erhält 6,6 g (26 % der Theorie) (E)-3-Amino-4-ethoxycarbonylmethoximino-2-pyrazolin-5-on mit einem Gehalt von 84 % (HPLC) und einem Schmelzpunkt von 152-158 ° C.

Herstellung des Ausgangsmaterials

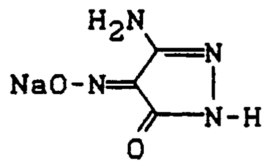

(VI-1)

164,1 g (1 Mol) des Natriumsalzes des (E)-2-Cyan-2-hydroximino-essigsäureethylesters werden mit 75 g (1,5 Mol) Hydrazinhydrat in 1,5 l Ethanol 3 Stunden unter Rückfluß erhitzt. Nach Abkühlung auf 5 ° C wird der Niederschlag abgesaugt, mit kaltem Ethanol gewaschen und unter vermindertem Druck über Sicapent getrocknet.

Man erhält 110 g (73 % der Theorie) des rotbraunen Natriumsalzes des (E)-3-Amino-4-hydroximino-2-pyrazolin-5-ons, das sich bei 280 ° C zersetzt.

In analoger Weise zu den in den Beispielen 1 bis 5 beschriebenen Methoden und unter Berücksichtigung der Angaben in den Beschreibungen zu den erfindungsgemäßen Verfahren, werden die in der nachfolgenden Tabelle 2 aufgeführten Endprodukte der Formel (I)

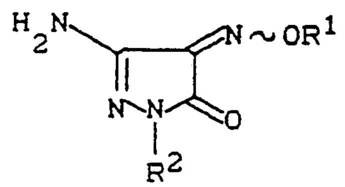

(I)

erhalten:

Tabelle 2

| Bsp.Nr. | $R^1$ | $R^2$ | physikalische Konstante/Fp. |
| --- | --- | --- | --- |
| 6 | 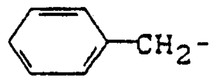 | 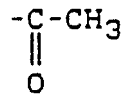 | 255-260° C |
| 7 | $CH_3$- | H | 193-195° C |
| 8 | 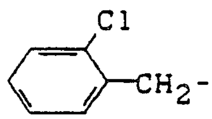 | H | >260° C |
| 9 | 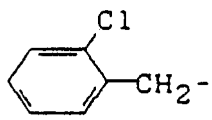 | $CH_3$- | amorph |
| 10 | $CH_3$- | $CH_3$- | 126° C |
| 11 | 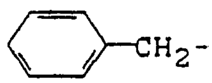 | 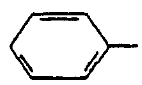 | 154-156° C |
| 12 | 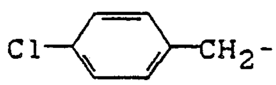 | $CH_3$- | 163-165° C |
| 13 | 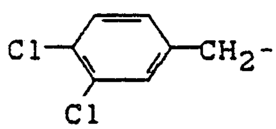 | H | >230° C |
| 14 | 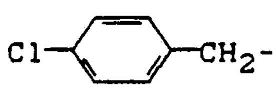 | H | 230-233° C |

Tabelle 2 - Fortsetzung

| Bsp.Nr. | $R^1$ | $R^2$ | physikalische Konstante/Fp. |
|---|---|---|---|
| 15 | 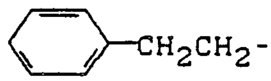 | H | 195-199° C |
| 16 | 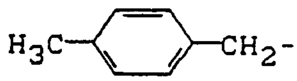 | H | 219-225° C |
| 17 | $CH_2$=CH-$CH_2$- | H | 136-139° C |
| 18 | 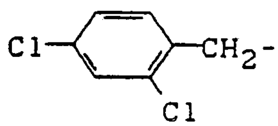 | H | 193-196° C |
| 19 | 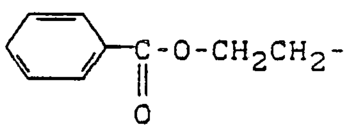 | H | 178-180° C |
| 20 | 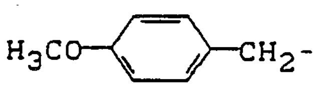 | H | 165-170° C |

## Anwendungsbeispiel

In dem folgenden Anwendungsbeispiel werden die nachstehend aufgeführten Verbindungen als Vergleichssubstanzen eingesetzt:

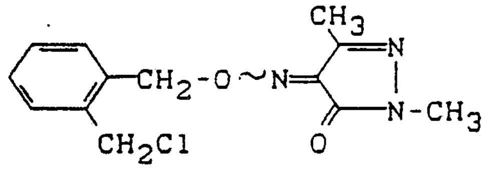

(A)

1,3-Dimethyl-4-(2-chlormethyl-benzyloximino)-2-pyrazolin-5-on

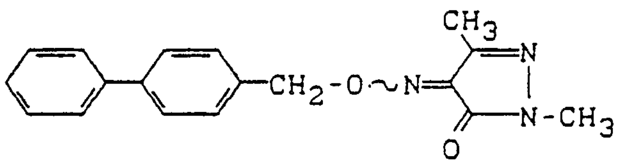

(B)

1,3-Dimethyl-4-(4-phenyl-benzyloximino)-2-pyrazolin-5-on

[Die Verbindungen (A) und (B) sind bekannt aus EP-OS 0 166 171]

Beispiel A

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

In diesem Test zeigen z.B. die erfindungsgemäßen Wirkstoffe 2, 3, 6, 11, 14, 15 und 16 bei einer Wirkstoffkonzentration von 0,025 Gew.-% in der Spritzbrühe einen höheren Wirkungsgrad als die Vergleichssubstanzen (A) und (B).

## Claims

1. Substituierte 3-Amino-2-pyrazolin-5-on-Derivate der allgemeinen Formel (I)

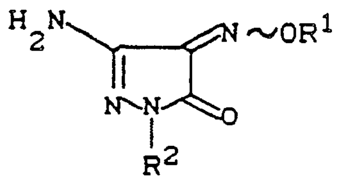

(I)

in welcher
$R^1$ für unsubstituiertes oder substituiertes Alkyl, unsubstituiertes oder substituiertes Alkenyl oder Alkinyl, unsubstituiertes oder substituiertes Cycloalkyl oder Cycloalkenyl steht,
$R^2$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl oder für einen der Reste $-CX-YR^3$, $-CXNR^4R^5$, $-\underset{\underset{O}{\|}}{C}-R^6$,
$-SO_2NR^4R^5$ oder $-SO_2R^7$ steht,
$R^3$ für unsubstituiertes oder substituiertes Alkyl oder unsubstituiertes oder substituiertes Alkenyl oder Alkinyl steht,
$R^4$ und $R^5$ gleich oder verschieden sind und für Wasserstoff, unsubstituiertes oder substituiertes Alkyl, unsubstituiertes oder substituiertes Alkenyl oder Alkinyl, unsubstituiertes oder substituiertes Aryl oder Heterocyclyl oder gemeinsam mit dem Stickstoffatom, an welches sie gebunden sind, für einen unsubstituierten oder substituierten heterocyclischen Ring stehen, der weitere Heteroatome enthalten kann,
$R^6$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl Alkoxycarbonyl oder Phenylcarbonyl steht,
$R^7$ für unsubstituiertes oder substituiertes Alkyl oder unsubstituiertes oder substituiertes Aryl steht und
X und Y gleich oder verschieden sind und für Sauerstoff oder Schwefel stehen.

2. Substituierte 3-Amino-2-pyrazolin-5-on-Derivate der Formel (I) gemäß Anspruch 1, wobei
$R^1$ für unsubstituiertes oder ein- bis fünffach, gleich oder verschieden substituiertes, geradkettiges oder verzweigtes Alkyl mit 1 bis 10 Kohlenstoffatomen steht, wobei folgende Substituenten infrage kommen: Fluor, Chlor, Brom, Iod, Cyano, -COOH, $-COOR^3$, $-CONR^4R^5$, $-OR^6$, $-SR^6$, Alkylcarbonyl mit 1 bis 9 Kohlenstoffatomen, unsubstituiertes oder ein- bis dreifach, gleich oder verschieden durch Halogen substituiertes Phenylcarbonyl, unsubstituiertes oder ein- bis fünffach, gleich oder verschieden substituiertes Phenyl, wobei als Phenylsubstituenten genannt seien; Halogen, Nitro, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen; Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil; weitere Alkylsubstituenten sind unsubstituiertes oder ein- bis fünffach, gleich oder verschieden durch Chlor oder Alkyl mit 1 bis 4 Kohlenstoffatomen substituiertes Cycloalkyl oder Cycloalkenyl mit 3 bis 6 Kohlenstoffatomen, unsubstituiertes oder ein- bis fünffach, gleich oder verschieden durch Halogen, Hydroxy, Oxo, Alkyl und Alkoxy mit 1 bis 4 Kohlenstoffatomen substituiertes 3- bis 6-gliedriges Heterocyclyl mit 1 bis 3 gleichen oder verschiedenen Heteroatomen, $R^1$ steht ferner für jeweils unsubstituiertes oder ein-bis dreifach, gleich oder verschieden substituiertes, geradkettiges oder verzweigtes Alkenyl oder Alkinyl mit 2 bis 6 Kohlenstoffatomen, wobei als Substituenten genannt seien: unsubstituiertes oder ein- bis fünffach, gleich oder verschieden durch Halogen, Alkyl und Alkoxy mit 1 bis 4 Kohlenstoffatomen substituiertes Phenyl; $R^1$ steht außerdem für jeweils unsubstituiertes oder ein- bis fünffach, gleich oder verschieden durch Chlor, Alkyl oder Alkoxy mit 1 bis 4 Kohlenstoffatomen substituiertes Cycloalkyl mit 3 bis 6 Kohlenstoffatomen oder Cycloalkenyl mit 5 bis 7 Kohlenstoffatomen;
$R^2$ für Wasserstoff, für unsubstituiertes oder ein- bis fünffach, gleich oder verschieden substituiertes, geradkettiges oder verzweigtes Alkyl mit 1 bis 6 Kohlenstoffatomen steht, wobei folgende Substituenten infrage kommen: Fluor, Chlor, Brom, Iod, Cyano, -COOH, $-COOR^3$, $-CONR^4R^5$, $-OR^6$, $-SR^6$, Alkylcarbonyl mit 1 bis 9 Kohlenstoffatomen, unsubstituiertes oder ein- bis dreifach, gleich oder verschieden durch Halogen substituiertes Phenylcarbonyl, unsubstituiertes oder ein- bis fünffach, gleich oder verschieden substituiertes Phenyl, wobei als Phenylsubstituenten genannt seien: Halogen, Nitro, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen, Amino Alkylamino und Dialkylamino mit jeweils 1

bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil; weitere Alkylsubstituenten sind unsubstituiertes oder ein- bis fünffach, gleich oder verschieden durch Chlor oder Alkyl mit 1 bis 4 Kohlenstoffatomen substituiertes Cycloalkyl mit 3 bis 6 Kohlenstoffatomen, unsubstituiertes oder ein- bis fünffach, gleich oder verschieden durch Halogen, Hydroxy, Oxo, Alkyl und Alkoxy mit 1 bis 4 Kohlenstoffatomen substituiertes 3- bis 6-gliedriges Heterocyclyl mit 1 bis 3 gleichen oder verschiedenen Heteroatomen; $R^2$ steht ferner für jeweils unsubstituiertes oder ein- bis dreifach, gleich oder verschieden substituiertes, geradkettiges oder verzweigtes Alkenyl oder Alkinyl mit 2 bis 6 Kohlenstoffatomen, wobei als Substituenten genannt seien: unsubstituiertes oder ein- bis fünffach, gleich oder verschieden durch Halogen und Alkyl mit 1 bis 4 Kohlenstoffatomen substituiertes Phenyl; $R^2$ steht ferner für jeweils unsubstituiertes oder ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen: $R^2$ steht ferner für unsubstituiertes oder ein- bis fünffach, gleich oder verschieden substituiertes Aryl mit 6 bis 12 Kohlenstoffatomen, wobei als Substituenten genannt seien: Halogen, Nitro, Alkyl oder Alkoxy mit jeweils 1 bis 4 Kohlenstoffatomen, Halogenalkyl oder Halogenalkoxy mit jeweils 1 oder 2 Kohlenstoffatomen im Alkylteil und 1 bis 5 gleichen oder verschiedenen Halogenatomen.

$R^2$ steht ferner für unsubstituiertes oder ein- bis fünffach, gleich oder verschieden durch Halogen, Hydroxy, Oxo, Alkyl und Alkoxy mit jeweils 1 bis 4 Kohlenstoffatomen substituiertes 3- bis 6-gliedriges Heterocyclyl mit 1 bis 3 gleichen oder verschiedenen Heteroatomen oder für einen der Reste $-CX-YR^3$, $-CXNR^4R^5$, $-CO-R^6$, $-SO_2-NR^4R^5$ oder $-SO_2R^7$, worin X und Y gleich oder verschieden sind und für Sauerstoff oder Schwefel stehen,

$R^3$ für unsubstituiertes oder ein- bis fünffach, gleich oder verschieden substituiertes, geradkettiges oder verzweigtes Alkyl mit 1 bis 6 Kohlenstoffatomen steht, wobei als Substituenten genannt seien: Halogen, unsubstituiertes oder ein- bis fünffach, gleich oder verschieden substituiertes Phenyl, wobei als Phenylsubstituenten Halogen, Nitro, Alkyl und Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen, Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil infrage kommen und Cycloalkyl oder Cycloalkenyl mit 3 bis 6 Kohlenstoffatomen; $R^3$ ferner für geradkettiges oder verzweigtes Alkenyl oder Alkinyl mit 3 bis 6 Kohlenstoffatomen,

$R^4$ und $R^5$ jeweils gleich oder verschieden sind und für Wasserstoff oder für unsubstituiertes oder gleich oder verschieden, ein- oder zweifach substituiertes Alkyl mit 1 bis 6 Kohlenstoffatomen steht, wobei folgende Substituenten infrage kommen: Alkoxy mit 1 bis 4 Kohlenstoffatomen, unsubstituiertes oder ein-bis fünffach, gleich oder verschieden durch Halogen, Nitro, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen, Amino, Alkylamino und Dialkylamino mit jeweils 1 bis 4 Kohlenstoffatomen in den einzelnen Alkylteilen, Aryloxy und Aralkoxy mit jeweils 6 bis 12 Kohlenstoffatomen im Arylteil und 1 bis 4 Kohlenstoffatomen im Alkoxyteil substituiertes Phenyl, für Cycloalkyl oder Cycloalkenyl mit 3 bis 6 Kohlenstoffatomen; $R^4$ und $R^5$ weiterhin für Alkenyl oder Alkinyl mit 3 bis 6 Kohlenstoffatomen, unsubstituiertes oder ein- bis fünffach, gleich oder verschieden substituiertes Phenyl steht, wobei als Phenylsubstituenten die oben bei $R^4$ und $R^5$ als Phenylsubstituenten genannten Substituenten infrage kommen oder für ein- bis dreifach, gleich oder verschieden substituiertes 3- bis 6-gliedriges Heterocyclyl mit 1 bis 3 gleichen oder verschiedenen Heteroatomen steht, wobei folgende Substituenten infrage kommen: Halogen, Hydroxy, Alkyl oder Alkoxy mit 1 bis 4 Kohlenstoffatomen und Oxo; ferner $R^4$ und $R^5$ gemeinsam mit dem Stickstoffatom, an welches sie gebunden sind, für einen ein- bis dreifach, gleich oder verschieden substituierten oder unsubstituierten 5- oder 6-gliedrigen Heterocyclus steht, der 1 oder 2 weitere, gleiche oder verschiedene Heteroatome enthalten kann, wobei als Substituenten Halogen, Hydroxy, Alkyl und Alkoxy mit 1 bis 4 Kohlenstoffatomen und Oxo infrage kommen,

$R^6$ für Wasserstoff, unsubstituiertes oder 1- bis 5-fach, gleich oder verschieden substituiertes, geradkettiges oder verzweigtes Alkyl mit 1 bis 6 Kohlenstoffatomen steht, wobei als Substituenten infrage kommen: Halogen, Alkoxy mit 1 bis 4 Kohlenstoffatomen, unsubstituiertes oder substituiertes Cycloalkyl mit 3 bis 6 Kohlenstoffatomen, ein- bis dreifach substituiertes oder unsubstituiertes Phenyl, wobei als ein bis 3 Substituenten die oben bei $R^4$ und $R^5$ als Phenylsubstituenten genannten infrage kommen; $R^6$ ferner für Alkenyl oder Alkinyl mit 2 bis 6 Kohlenstoffatomen, Cycloalkyl mit 3 bis 6 Kohlenstoffatomen, ein- bis dreifach, gleich oder verschieden substituiertes oder unsubstituiertes Phenyl steht, wobei als Phenylsubstituenten die oben bei $R^4$ und $R^5$ als Phenylsubstituenten genannten Substituenten infrage kommen, unsubstituiertes oder ein-bis dreifach, gleich oder verschieden substituiertes 3- bis 6-gliedriges Heterocyclyl mit 1 bis 3 gleichen oder verschiedenen Heteroatomen steht, wobei als Substituenten Halogen, Hydroxy,

Alkyl oder Alkoxy mit jeweils 1 bis 4 Kohlenstoffatomen oder Oxo infrage kommen und weiterhin für Alkoxycarbonyl mit 1 oder 2 Kohlenstoffatomen oder Phenylcarbonyl,

$R^7$ für unsubstituiertes oder ein- bis fünffach durch Chlor substituiertes Alkyl mit 1 bis 6 Kohlenstoffatomen oder für unsubstituiertes oder ein- bis dreifach, gleich oder verschieden substituiertes Phenyl steht, wobei als Phenylsubstituenten die oben bei $R^4$ und $R^5$ als Phenylsubstituenten genannten Substituenten infrage kommen.

3. Substituierte 3-Amino-2-pyrazolin-5-on-Derivate der allgemeinen Formel (I) gemäß Anspruch 1, worin $R^1$ für unsubstituiertes Alkyl mit 1 bis 8 Kohlenstoffatomen oder ein- bis dreifach, gleich oder verschieden substituiertes, geradkettiges oder verzweigtes Alkyl mit 1 bis 5 Kohlenstoffatomen steht, wobei folgende Substituenten infrage kommen: Fluor, Chlor, Brom, Iod, Cyano, -COOH, $-COOR^3$, $-CONR^4R^5$, $-OR^6$, $-SR^6$, Alkylcarbonyl mit 1 bis 7 Kohlenstoffatomen, unsubstituiertes oder ein- oder zweifach durch Halogen substituiertes Phenylcarbonyl, unsubstituiertes oder ein- bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylmethoxy substituiertes Phenyl, weitere Alkylsubstituenten sind jeweils unsubstituiertes oder ein- bis dreifach durch Methyl oder Chlor substituiertes Cyclopropyl, Cyclopentyl, Cyclohexyl, Cyclopentenyl oder Cyclohexenyl und unsubstituiertes oder einfach bis dreifach, gleich oder verschieden durch Fluor, Chlor, Methyl, Methoxy, oder Hydroxy substituierte Heterocyclen der Formeln

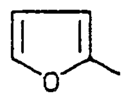 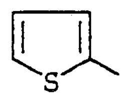 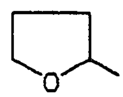 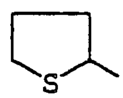 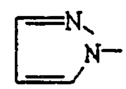

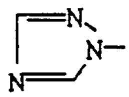 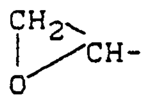 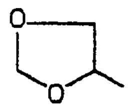 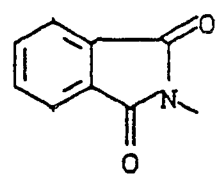

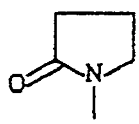 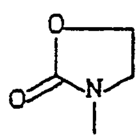 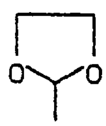 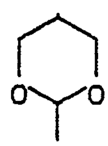 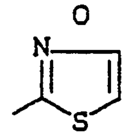 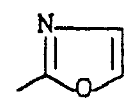

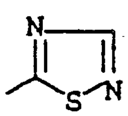 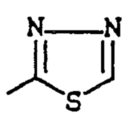 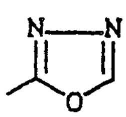 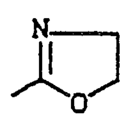 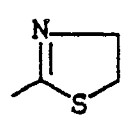 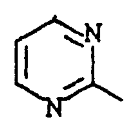

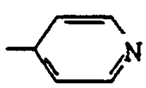 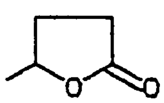 ,

$R^1$ ferner für ein- oder zweifach substituiertes Allyl oder Propargyl steht, wobei als Substituenten unsubstituiertes oder ein- bis dreifach, gleich oder verschieden durch Fluor, Chlor, Methyl, Methoxy substituiertes Phenyl genannt seien, $R^1$ weiterhin für jeweils unsubstituiertes oder ein- bis dreifach durch Chlor, Methyl oder Methoxy substituiertes Cyclopropyl, Cyclopentyl, Cyclohexyl, Cyclopentenyl oder Cyclohexenyl steht;

$R^2$ für Wasserstoff, für unsubstituiertes oder ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, -COOH, $-COOR^3$, $-CONR^4R^5$, $-OR^6$, $SR^6$, Alkylcarbonyl mit 1 bis 7 Kohlenstoffatomen, unsubstituiertes oder ein- bis dreifach, gleich oder verschieden durch Halogen substituiertes Phenylcarbonyl, unsubstituiertes oder ein- bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylalkoxy mit 1 oder 2 Kohlenstoffatomen im Alkoxyteil substituiertes Phenyl; weitere Alkylsubstituenten sind jeweils unsubstituiertes oder ein- bis dreifach durch Methyl oder Chlor substituiertes Cyclopropyl, Cyclopentyl, Cyclohexyl, Cyclopentenyl oder Cyclohexenyl oder unsubstituierte oder einfach bis dreifach, gleich oder verschieden durch Fluor, Chlor, Methyl, Methoxy oder

Hydroxy substituierte Heterocyclen der Formeln

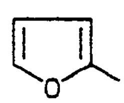 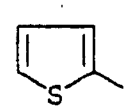 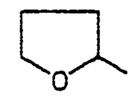 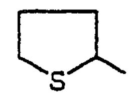 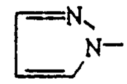

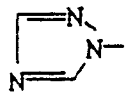 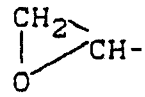 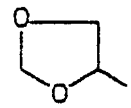 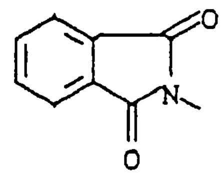

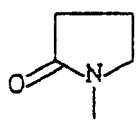 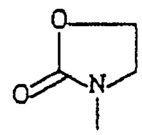 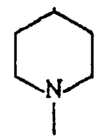 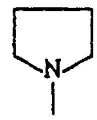 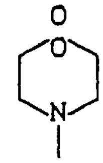 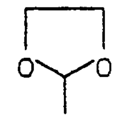

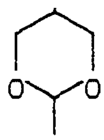 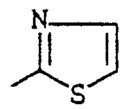 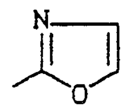 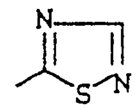 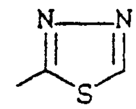

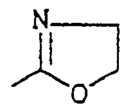 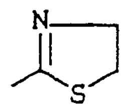 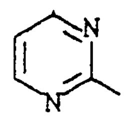 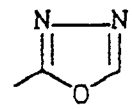

$R^2$ steht ferner für ein- oder zweifach, gleich oder verschieden substituiertes Allyl oder Propargyl, wobei als Substituenten unsubstituiertes oder ein-bis dreifach, gleich oder verschieden durch Fluor, Chlor, Methyl oder Methoxy substituiertes Phenyl genannt seien, weiterhin steht $R^2$ für jeweils unsubstituiertes oder ein-bis dreifach durch Methyl oder Methoxy substituiertes Cyclopropyl, Cyclopentyl, Cyclohexyl, Cyclopentenyl oder Cyclohexenyl,
$R^2$ steht ferner für unsubstituiertes oder ein- bis dreifach, gleich oder verschieden substituiertes Phenyl, wobei als Substituenten genannt seien: Fluor, Chlor, Nitro, Methyl, Methoxy, Trifluormethyl, Trifluormethoxy,
$R^2$ steht ferner für unsubstituiertes oder ein- bis dreifach, gleich oder verschieden durch Fluor, Chlor, Hydroxy, Oxo, Methyl oder Methoxy substituierte Heterocyclen der Formeln

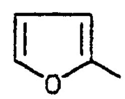 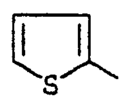 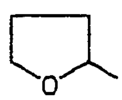 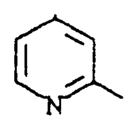 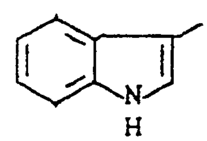

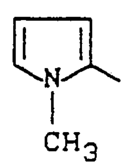 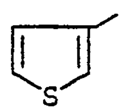 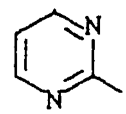 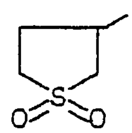

oder für einen der Reste $-CX-YR^3$, $-CXNR^4R^5$, $-COR^6$, $-SO_2-NR^4R^5$ oder $-SO_2R^7$ steht,
worin
X und Y gleich oder verschieden sind und für Sauerstoff oder Schwefel stehen,
$R^3$ für unsubstituiertes oder ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, unsubstituiertes oder einfach bis dreifach, gleich oder verschieden durch Fluor, Chlor, Nitro, Methyl, Methoxy, Trifluormethyl, Trifluormethoxy, Amino, Methylamino, Dimethylamino, Phenyloxy und Phenylalkoxy mit 1 oder 2 Kohlenstoffatomen im Alkylteil substituiertes Phenyl und jeweils unsubstituiertes oder ein- bis dreifach durch Methyl substituiertes Cyclopropyl, Cyclopentyl, Cyclohexyl, Cyclopentenyl, Cyclohexenyl; $R^3$ weiterhin für Allyl oder Propargyl steht,
$R^4$ und $R^5$ gleich oder verschieden sind und für Wasserstoff oder für unsubstituiertes oder gleich oder

verschieden, ein- oder zweifach substituiertes Alkyl mit 1 bis 4 Kohlenstoffatomen stehen, wobei folgende Substituenten infrage kommen: Methoxy, Ethoxy, unsubstituiertes oder ein- bis dreifach, gleich oder verschieden durch Fluor, Chlor, Brom, Iod, Nitro, Methyl und Methoxy, Trifluormethyl, Trifluormethoxy, Amino, Methylamino, Dimethylamino, Phenoxy, Phenylmethoxy substituiertes Phenyl; $R^4$ und $R^5$ weiterhin für Allyl, Propargyl, Cyclopropyl, Cyclopentyl, Cyclohexyl, Cyclopentenyl, Cyclohexenyl, unsubstituiertes oder ein- bis dreifach, gleich oder verschieden substituiertes Phenyl stehen, wobei als Phenylsubstituenten die oben bei $R^4$ und $R^5$ als Phenylsubstituenten genannten Substituenten infrage kommen, oder ferner für einfach bis dreifach, gleich oder verschieden durch Fluor, Chlor, Methyl, Methoxy, Hydroxy oder Oxo substituierte Heterocyclen der Formeln

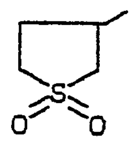 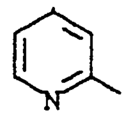 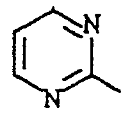 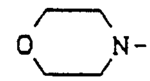 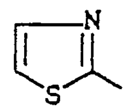

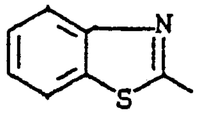 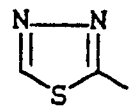 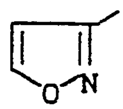 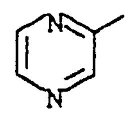 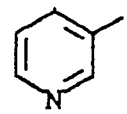

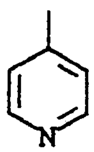 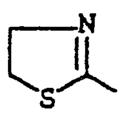 stehen,

oder

$R^4$ und $R^5$ gemeinsam mit dem Stickstoffatom, an welches sie gebunden sind, für jeweils unsubstituiertes oder substituiertes Oxazolin, Pyrrolidin, Imidazolin, Piperazin, Morpholin, Thiomorpholin, 1,3-Oxazan oder 1,3-Diazan stehen, wobei als Substituenten Fluor, Chlor, Brom, Iod, Hydroxy, Methyl, Methoxy und Oxo infrage kommen,

$R^6$ für Wasserstoff, für unsubstituiertes oder ein- bis dreifach, gleich oder verschieden substituiertes geradkettiges oder verzweigtes Alkyl mit 1 bis 4 Kohlenstoffatomen steht, wobei als Substituenten infrage kommen: Fluor, Chlor, Brom, Iod, Methoxy, Cyclopropyl, Cyclopentyl, Cyclohexyl, ein- oder zweifach, gleich oder verschieden substituiertes oder unsubstituiertes Phenyl, wobei als Phenylsubstituenten die oben bei $R^4$ und $R^5$ als Phenylsubstituenten genannten infrage kommen; ferner für Allyl oder Propargyl, Cyclopropyl, Cyclopentyl, Cyclohexyl oder für ein- oder zweifach, gleich oder verschieden substituiertes oder unsubstituiertes Phenyl steht, wobei als Phenylsubstituenten die oben bei $R^4$ und $R^5$ als Phenylsubstituenten genannten Substituenten infrage kommen; weiterhin für unsubstituiertes oder 1- bis 3-fach, gleich oder verschieden durch Fluor, Chlor, Brom, Iod, Hydroxy, Oxo, Methyl, Methoxy substituierte Heterocyclen der Formeln

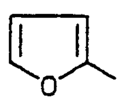 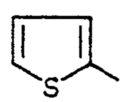 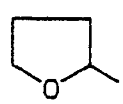 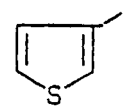 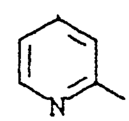

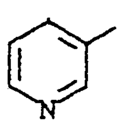 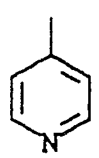 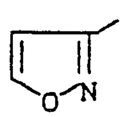 steht

und weiterhin für Methoxycarbonyl, Ethoxycarbonyl oder Phenylcarbonyl steht,

$R^7$ für unsubstituiertes oder ein- bis dreifach durch Chlor substituiertes Alkyl mit 1 bis 4 Kohlenstoffatomen oder für unsubstituiertes oder ein- bis dreifach, gleich oder verschieden substituiertes Phenyl steht, wobei als Phenylsubstituenten die oben bei $R^4$ und $R^5$ als Phenylsubstituenten genannten Substituenten infrage kommen.

4. Substituierte 3-Amino-2-pyrazolin-5-on-Derivate gemäß Anspruch 1, worin in der Formel (I)

$R^1$ für unsubstituiertes Alkyl mit 1 bis 8 Kohlenstoffatomen, oder für ein- oder zweifach, gleich oder

verschieden substituiertes Methyl, Ethyl, n- oder i-Propyl steht, wobei folgende Substituenten infrage kommen: Fluor, Cyano, -COOH,
$-COOR^3$, $-CONR^4R^5$, $-OR^6$, $-SR^6$, Alkylcarbonyl mit 1 bis 5 Kohlenstoffatomen, unsubstituiertes oder ein- oder zweifach, gleich oder verschieden durch Fluor, Chlor, Brom substituier tes Phenylcarbonyl, unsubstituiertes oder ein- bis dreifach, gleich oder verschieden durch Fluor, Chlor, Nitro, Methyl, Methoxy, Trifluormethyl, Methylamino, Dimethylamino und Phenylmethoxy substituiertes Phenyl; weitere Alkylsubstituenten sind jeweils unsubstituiertes oder ein-oder zweifach durch Methyl oder Chlor substituierte Cyclopropyl, Cyclohexyl oder Cyclohexenyl oder unsubstituiertes oder einfach bis dreifach durch Methyl substituierte Heterocyclen der Formeln

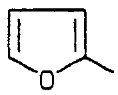 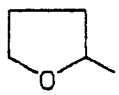 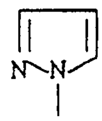 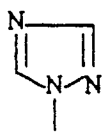 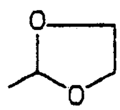 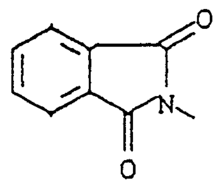 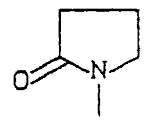

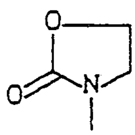 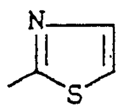 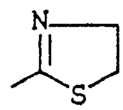 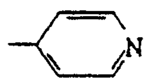 und 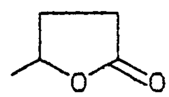

stehen,
$R^1$ ferner für durch Phenyl substituiertes Allyl oder Propargyl oder für jeweils unsubstituiertes oder ein- oder zweifach durch Methyl oder Chlor substituiertes Cyclohexyl, Cyclopropyl oder Cyclohexenyl steht,
$R^2$ für Wasserstoff, für unsubstituiertes oder ein- bis dreifach, gleich oder verschieden substituiertes Methyl oder Ethyl steht, wobei folgende Substituenten infrage kommen: Fluor, Chlor, Cyano, -COOH, $-COOR^3$, $-CONR^4R^5$, $-OR^6$, $-SR^6$, Alkylcarbonyl mit 1 bis 5 Kohlenstoffatomen, unsubstituiertes oder ein-oder zweifach, gleich oder verschieden durch Fluor, Chlor oder Brom substituiertes Phenylcarbonyl, Phenyl, Cyclopropyl, 1,3-Dioxolan-2-yl oder 1,2,4-Triazol-1-yl; $R^2$ weiterhin für Allyl, Propargyl, für Cyclohexyl, unsubstituiertes oder ein- oder zweifach, gleich oder verschieden durch Chlor, Methyl oder Methoxy substituiertes Phenyl oder für einen der Reste
$-CX-YR^3$, $-CXNR^4R^5$, $-COR^6$, $-SO_2-NR^4R^5$, $-SO_2R^7$ oder

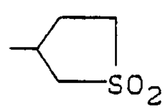

steht,
worin
X und Y gleich oder verschieden sind und für Sauerstoff oder Schwefel stehen,
$R^3$ für Methyl, Ethyl, n- und i-Propyl, n-, i-, s- oder t-Butyl steht,
$R^4$ für Wasserstoff, Methyl, Ethyl, n- oder i-Propyl, n-, i-, s- oder t-Butyl oder Phenyl steht,
$R^5$ für Wasserstoff, Methyl, Ethyl, n- oder i-Propyl, n-, i-, s- oder t-Butyl oder Phenyl steht,
$R^6$ für Wasserstoff, unsubstituiertes oder ein-bis dreifach durch Fluor, Chlor, Methoxy und Phenyl substituiertes Methyl, Ethyl, n- oder i-Propyl oder n-, i-, s- oder t-Butyl steht, $R^6$ fernerfür Phenyl, Methoxycarbonyl, Ethoxycarbonyl oder Phenylcarbonyl steht und
$R^7$ für Methyl, Ethyl, n- oder i-Propyl oder n-, i-, s-oder t-Butyl oder für unsubstituiertes oder ein- oder zweifach durch Methyl substituiertes Phenyl steht.

5. 3-Amino-2-pyrazolin-5-on-Derivate der Formel (I) gemäß Anspruch 1, worin
$R^1$ für unsubstituiertes, geradkettiges oder verzweigtes Alkyl mit 1 bis 6 Kohlenstoffatomen, für ein- oder zweifach, gleich oder verschieden substituiertes Methyl, Ethyl, n-oder i-Propyl steht, wobei als Substituenten genannt seien: Fluor, Cyano, -COOH, $-COOR^3$, $-CONR^4R^5$, $-OR^6$, $-SR^6$, Alkylcarbonyl mit 1 bis 5 Kohlenstoffatomen im Alkylteil, unsubstituiertes oder ein- oder zweifach, gleich oder verschieden durch Brom substituiertes Phenylcarbonyl, unsubstituiertes oder ein- bis dreifach, gleich oder verschieden durch Chlor, Methyl, Methoxy, Nitro, Dimethylamino, Benzyloxy und Trifluormethyl substituiertes Phenyl, unsubstituiertes Cyclopropyl, Cyclohexyl oder Cyclohexenyl, ein- oder zweifach, gleich oder verschieden durch

Chlor und Methyl substitu iertes Cyclopropyl, unsubstituierte oder ein- bis dreifach durch Methyl, substituierte Heterocyclen der Formel

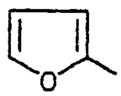 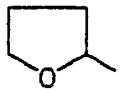 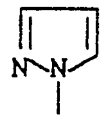 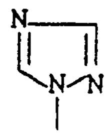 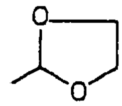 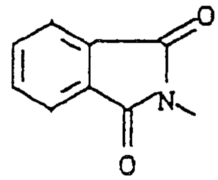 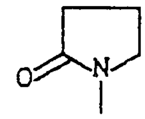

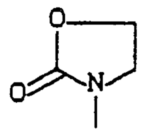 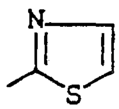 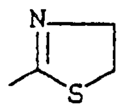 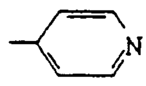 und 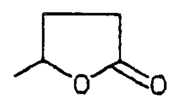

$R^1$ steht weiterhin für Allyl, Phenylallyl, Propargyl, Cyclohexyl oder Cyclohexenyl,

$R^2$ steht für Wasserstoff, geradkettiges oder verzweigtes Alkyl mit 1 bis 4 Kohlenstoffatomen, Alkylcarbonyl mit 1 bis 3 Kohlenstoffatomen im geradkettigen oder verzweigten Alkylteil, Alkylaminocarbonyl mit 1 oder 2 Kohlenstoffatomen im geradkettigen oder verzweigten Alkylteil, für einfach durch Alkoxy oder Alkoxycarbonyl mit 1 bis 3 Kohlenstoffatomen im Alkoxyteil, Cyano, Hydroxy, Aminocarbonyl, Phenyl, -COOH, Phenylcarbonyl, Cyclopropyl, Triazolyl oder Dioxolanyl substituiertes geradkettiges oder verzweigtes Alkyl mit 1 bis 3 Kohlenstoffatomen, Halogenalkyl mit 1 bis 3 Kohlenstoffatomen und 1 bis 5 gleichen oder verschiedenen Halogenatomen, wie Fluor und Chlor, Phenylsulfonyl, Phenyl oder ein- bis dreifach, gleich oder verschieden durch Chlor und Methyl substituiertes Phenyl, Alkylthiocarbonyl mit 1 bis 3 Kohlenstoffatomen im geradkettigen oder verzweigten Alkythioteil, Alkenyl oder Alkinyl mit 3 oder 4 Kohlenstoffatomen.

$R^3$ für Methyl, Ethyl, n- und i-Propyl, n-, i-, s- oder t-Butyl steht,

$R^4$ für Wasserstoff, Methyl, Ethyl, n- oder i-Propyl, n-, i-, s- oder t-Butyl oder Phenyl steht,

$R^5$ für Wasserstoff, Methyl, Ethyl, n- oder i-Propyl, n-, i-, s- oder t-Butyl oder Phenyl steht,

$R^6$ für Wasserstoff, unsubstituiertes oder ein-bis dreifach durch Fluor, Chlor, Methoxy und Phenyl substituiertes Methyl, Ethyl, n- oder i-Propyl oder n-, i-, s- oder t-Butyl steht, $R^6$ fernerfür Phenyl, Methoxycarbonyl, Ethoxycarbonyl oder Phenylcarbonyl steht.

6. Verfahren zur Herstellung von 3-Amino-2-pyrazolin-5-on-Derivaten der allgemeinen Formel (I),

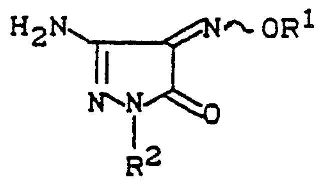 (I)

in welcher

$R^1$ für unsubstituiertes oder substituiertes Alkyl, unsubstituiertes oder substituiertes Alkenyl oder Alkinyl, unsubstituiertes oder substituiertes Cycloalkyl oder Cycloalkenyl steht,

$R^2$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl oder für einen der Reste

$-CX-YR^3$, $-CXNR^4R^5$, $-\underset{\underset{O}{\|}}{C}-R^6$,

$-SO_2NR^4R^5$ oder $-SO_2R^7$

steht,

$R^3$ für unsubstituiertes oder substituiertes Alkyl oder unsubstituiertes oder substituiertes Alkenyl oder Alkinyl steht,

$R^4$ und $R^5$ gleich oder verschieden sind und für Wasserstoff, unsubstituiertes oder substituiertes Alkyl, unsubstituiertes oder substi tuiertes Alkenyl oder Alkinyl, unsubstituiertes oder substituiertes Aryl oder Heterocyclyl oder gemeinsam mit dem Stickstoffatom, an welches sie gebunden sind, für einen unsubstituierten oder substituierten heterocyclischen Ring stehen, der weitere Heteroatome enthalten kann,

$R^6$ für Wasserstoff, unsubstituiertes oder substituiertes Alkyl, unsubstituiertes oder substituiertes Alkenyl oder Alkinyl, unsubstituiertes oder substituiertes Cycloalkyl oder Cycloalkenyl, unsubstituiertes oder substituiertes Aryl, unsubstituiertes oder substituiertes Heterocyclyl, Alkoxycarbonyl oder Phenylcarbonyl steht,
$R^7$ für unsubstituiertes oder substituiertes Alkyl oder unsubstituiertes oder substituiertes Aryl steht und
X und Y gleich oder verschieden sind und für Sauerstoff oder Schwefel stehen, deren geometrischen Isomere und Isomerengemische, dadurch gekennzeichnet, daß man

(a) 2-Cyan-2-oximino-essigsäure-Derivate der Formel (II)

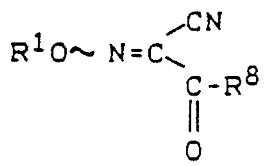

(II)

in welcher
$R^1$ die oben angegebene Bedeutung hat und
$R^8$ für niederes Alkoxy oder Halogen steht,
mit Hydrazinen der Formel (III)

$H_2N$-$NH$-$R^2$ (III)

in welcher
$R^2$ die oben angegebene Bedeutung hat,
gegebenenfalls in Gegenwart eines Verdünnungsmittels gegebenenfalls in Gegenwart einer Base und gegebenenfalls in Gegenwart eines Katalysators umsetzt oder daß man

(b) die nach Verfahren (a) erhältlichen 4-Oximino-2-pyrazolin-5-on-Derivate der allgemeinen Formel (Ia)

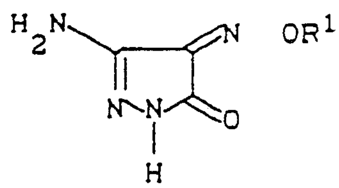

(Ia)

in welcher
$R^1$ die oben angegebene Bedeutung hat, (b1) mit Mitteln der Formel (IV)

$R^{2-1}$-A (IV)

in welcher
$R^{2-1}$ für unsubstituiertes oder substituiertes Alkyl, unsubstituiertes oder substituiertes Alkenyl oder Alkinyl, unsubstituiertes oder substituiertes Cycloalkyl unsubstituiertes oder substituiertes Cycloalkenyl oder substituiertes oder unsubstituiertes Heterocyclyl steht und
A für eine elektronenanziehende Abgangsgruppe steht
oder
(b2) mit Acylierungsmitteln der Formel (Va)

$R^{2-2}$-Z (Va)

in welcher
$R^{2-2}$ für einen der Reste
$-CX-YR^3$, $-CXNR^4R^5$, $-\underset{\underset{O}{\|}}{C}-R^6$,
$-SO_2NR^4R^5$ oder $-SO_2R^7$ steht,
Z für eine übliche Abgangsgruppe steht
und
$R^3$, $R^4$, $R^5$, $R^6$, $R^7$, X und Y die oben angegebene Bedeutung haben
oder
(b3) mit Acylierungsmitteln der Formel (Vb)

$R^{2-3}$-N=C=X (Vb)

in welcher

$R^{2-3}$ für unsubstituiertes oder substituiertes SAlkyl, unsubstituiertes oder substituiertes Alkenyl oder Alkinyl, unsubstituiertes oder substituiertes Cycloalkyl oder Cycloalkenyl, unsubstituiertes oder substituiertes Aryl oder substituiertes oder unsubstituiertes Heterocyclyl steht und
X für Sauerstoff oder Schwefel steht,
gegebenenfalls in Gegenwart eines Verdünnungsmittels, gegebenenfalls in Gegenwart einer Base und gegebenenfalls in Gegenwart eines Katalysators umsetzt
oder daß man
(c) 4-Oximino-2-pyrazolin-5-on-Derivate der Formel (VI)

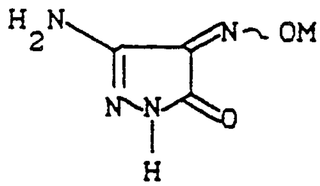

(VI)

in welcher
M für Wasserstoff oder ein Alkalimetallkation steht,
mit Alkylierungsmitteln der Formel (VII)
$R^1$-A (VII)
in welcher
$R^1$ die oben angegebene Bedeutung hat und
A für eine elektronenanziehende Abgangsgruppe steht,
gegebenenfalls in Gegenwart eines Verdünnungsmittels und gegebenenfalls in Gegenwart eines Katalysators umsetzt.

7. Schädlingsbekämpfungsmittel, gekennzeichnet durch einen Gehalt an mindestens einem substituierten 3-Amino-2-pyrazolin-5-on-Derivat der Formel (I) nach den Ansprüchen 1 und 6.

8. Verwendung von substituierten 3-Amino-2-pyrazolin-5-on-Derivaten der Formel (I) nach den Ansprüchen 1 und 6 zur Bekämpfung von Schädlingen.

9. Verfahren zur Bekämpfung von Schädlingen, dadurch gekennzeichnet, daß man substituierte 3-Amino-2-pyrazolin-5-on-Derivate der Formel (I) nach den Ansprüchen 1 und 6 auf Schädlinge und/oder ihren Lebensraum einwirken läßt.

10. Verfahren zur Herstellung von Schädlingsbekämpfungsmitteln, dadurch gekenzeichnet, daß man substituierte 3-Amino-2-pyrazolin-5-on-Derivate der Formel (I) nach den Ansprüchen 1 und 6 mit Streckmitteln und/oder oberflächenaktiven Miteln vermischt.

Europäisches Patentamt

# EUROPÄISCHER RECHERCHENBERICHT

Nummer der Anmeldung

EP 90 10 2526

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 166 171 (BAYER AG)<br>* Ansprüche 1,4-8 * | 1,6-10 | C 07 D 231/46<br>A 01 N 43/56 |
| A | DE-A-3 628 901 (BAYER AG)<br>* Ansprüche 1,4-8 * | 1,6-10 | |
| A | EP-A-0 225 472 (BAYER AG)<br>* Ansprüche 1,6-10 * | 1,6-10 | |
| A | EP-A-0 212 360 (BAYER AG)<br>* Ansprüche 1,4-8 * | 1,6-10 | |
| A | EP-A-0 294 668 (BAYER AG)<br>* Seite 3, Formel (II) *; & DE - A - 37 19226 (Kat. D) | 6 | |

RECHERCHIERTE SACHGEBIETE (Int. Cl.5)

A 01 N 43/00
C 07 D 231/00

Der vorliegende Recherchenbericht wurde für alle Patentansprüche erstellt

| Recherchenort | Abschlußdatum der Recherche | Prüfer |
|---|---|---|
| BERLIN | 21-05-1990 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)